# EUROPEAN PATENT APPLICATION

(11) **EP 4 273 175 A1**
(43) Date of publication of application: **08.11.2023**
(21) Application number: 21924358.1
(22) Date of filing: 07.12.2021
(51) Int. Cl.: C07K 19/00, C12N 15/62, C12N 5/10, C12N 15/867, A61K 39/00, A61P 35/00, A61P 35/02

(54) **NOVEL CHIMERIC ANTIGEN RECEPTOR AND USE THEREOF**

(30) Priority: 03.02.2021 CN 202110147730
(71) Applicant: Bioheng Therapeutics Limited, Grand Cayman KY1-1209 (KY)
(72) Inventor: ZHOU, Yali, Nanjing, Jiangsu 210061 (CN); CHEN, Gong, Nanjing, Jiangsu 210061 (CN); GUO, Tingting, Nanjing, Jiangsu 210061 (CN); JIANG, Xiaoyan, Nanjing, Jiangsu 210061 (CN); REN, Jiangtao, Nanjing, Jiangsu 210061 (CN); HE, Xiaohong, Nanjing, Jiangsu 210061 (CN); WANG, Yanbin, Nanjing, Jiangsu 210061 (CN); HAN, Lu, Nanjing, Jiangsu 210061 (CN)
(74) Representative: Comoglio, Elena
(86) International application number: PCT/CN2021/135963
(87) International publication number: WO 2022/166365

(57) **Abstract**

Provided is a chimeric antigen receptor, comprising an antigen binding region, a transmembrane domain and an intracellular signaling region. The antigen binding region comprises an antibody specifically targeting CD7, and the intracellular signaling region consists of a co-stimulatory domain, a primary signal transduction domain, and a γC chain or intracellular region thereof. Also provided are an engineered immune cell comprising the chimeric antigen receptor and a pharmaceutical composition thereof, and use of the engineered immune cell/pharmaceutical composition for treating cancers.

## Description

### Technical Field

The present disclosure belongs to the field of immunotherapy. More specifically, the present disclosure relates to a novel chimeric antigen receptor targeting CD7 and use thereof in the treatment of diseases.

### Background Art

CD7 is a cell surface glycoprotein with a molecular weight of about 40 kDa and belongs to the immunoglobulin superfamily. CD7 is expressed on most T cells, NK cells, myeloid cells, T cell acute lymphoblastic leukemia/lymphoma, acute myelocytic leukemia and chronic myelocytic leukemia. It has been reported that the CD7 molecule acts as a co-stimulatory signal during T cell activation through binding to its ligand K12/SECTM1. Moreover, disruption of the CD7 molecule in mouse T progenitor cells still resulted in normal T cell development and homeostasis, suggesting that CD7 does not appear to have critical effects on T cell development and function, making it a very suitable therapeutic target for the treatment of acute lymphoblastic leukemia (ALL). Indeed, CD7 has been extensively studied as a target of cytotoxic molecules for the treatment of leukemia and lymphoma.

Currently, CAR-T therapy targeting CD7 is very limited. Therefore, the present disclosure aims to provide a highly efficient CAR cell therapy targeting CD7.

### Summary

In a first aspect, the present disclosure provides a novel chimeric antigen receptor, comprising an antigen binding region, a transmembrane domain and an intracellular signaling region, wherein the antigen binding region comprises an antibody specifically targeting CD7, and the intracellular signaling region consists of a co-stimulatory domain, a primary signaling domain, and a γc chain or intracellular region thereof.

In a preferred embodiment, the amino acid sequence of the γc chain is set forth in SEQ ID NO: 14; and the amino acid sequence of its intracellular region is set forth in SEQ ID NO: 16.

In an embodiment, the antibody is selected from the group consisting of IgG, Fab, Fab', F(ab')₂, Fd, Fd', Fv, scFv, sdFv, linear antibody, single domain antibody, nanobody and diabody, preferably selected from the group consisting of Fab, scFv, single domain antibody and nanobody, most preferably scFv.

In an embodiment, the antibody is selected from the group consisting of a monoclonal antibody, a polyclonal antibody, a recombinant antibody, a human antibody, a humanized antibody, a murine antibody and a chimeric antibody.

In an embodiment, the transmembrane domain is a transmembrane domain of a protein selected from the group consisting of : TCRα chain, TCRβ chain, TCRγ chain, TCRδ chain, CD3ζ subunit, CD3ε subunit, CD3γ subunit, CD3δ subunit, CD45, CD4, CD5, CD8α, CD9, CD16, CD22, CD33, CD28, CD37, CD64, CD80, CD86, CD134, CD137, and CD154. Preferably, the transmembrane domain is a transmembrane domain of a protein selected from the group consisting of CD8α, CD4, CD28 and CD278.

In an embodiment, the co-stimulatory domain is one or more co-stimulatory signaling domains of a protein selected from the group consisting of: TLR1, TLR2, TLR3, TLR4, TLR5, TLR6, TLR7, TLR8, TLR9, TLR10, CARD1 1, CD2, CD7, CD8, CD18 (LFA-1), CD27, CD28, CD30, CD40, CD54 (ICAM), CD83, CD134 (OX40), CD137 (4-1BB), CD270 (HVEM), CD272 (BTLA), CD276 (B7-H3), CD278 (ICOS), CD357 (GITR), DAP10, LAT, NKG2C, SLP76, PD-1, LIGHT, TRIM, CD94, LTB, and ZAP70. Preferably, the co-stimulatory domain is a co-stimulatory signaling domain of CD27, CD28, CD134, CD137 or CD278.

In an embodiment, the primary signaling domain is a signaling domain of a protein selected from the group consisting of: FcRγ, FcRβ, CD3γ, CD3δ, CD3ε, CD3ζ, CD22, CD79a, CD79b, and CD66d. Preferably, the primary signaling domain comprises the signaling domain of CD3ζ.

In an embodiment, the antigen binding region of the chimeric antigen receptor further comprises an antibody targeting a second antigen, wherein the second antigen is selected from the group consisting of: TSHR, CD19, CD123, CD22, BAFF-R, CD30, CD171, CS-1, CLL-1, CD33, EGFRvIII, GD2, GD3, BCMA, GPRC5D, Tn Ag, PSMA, ROR1, FLT3, FAP, TAG72, CD38 , CD44v6, CEA, EPCAM, B7H3, KIT, IL-13Ra2, mesothelin, IL-1 1Ra, PSCA, PRSS21, VEGFR2, LewisY, CD24, PDGFR-β, SSEA-4, CD20, Folate receptor α, ERBB2 (Her2/neu), MUC1, EGFR, NCAM, Claudin18.2, Prostase, PAP, ELF2M, Ephrin B2, IGF-I receptor, CAlX, LMP2, gploo, bcr-ab1, tyrosinase, EphA2, Fucosyl GM1, sLe, GM3, TGS5, HMWMAA, o-acetyl-GD2, Folate receptor β, TEM1/CD248, TEM7R, CLDN6, GPRC5D, CXORF61, CD97, CD 179a, ALK, polysialic acid, PLAC1, GloboH, NY-BR-1, UPK2, HAVCR1, ADRB3, PANX3, GPR20, LY6K, OR51E2, TARP, WT1, NY-ESO-1, LAGE-1a, MAGE-A1, legumain, HPV E6, E7, MAGE A1, ETV6-AML, sperm protein 17, XAGE1, Tie 2, MAD-CT-1, MAD-CT-2, Fos associated antigen 1, p53, p53 mutant, prostate specific protein, survivin and telomerase, PCTA-1/Galectin 8, MelanA/MART1, Ras mutant, hTERT, sarcoma translocation breakpoint, ML-IAP, ERG (TMPRSS2 ETS fusion gene), NA17, PAX3, androgen receptor, Cyclin B1, MYCN, RhoC, TRP-2, CYP1B 1, BORIS, SART3, PAX5, OY-TES 1, LCK, AKAP-4, SSX2, RAGE-1, human telomerase reverse transcriptase, RU1, RU2, intestinal tract carboxylesterase, mut hsp70-2, CD79a, CD79b, CD72, LAIR1, FCAR, LILRA2, CD300LF, CLEC12A, BST2, EMR2, LY75, GPC3, FCRL5, IGLL1, PD1, PDL1, PDL2, TGFβ, APRIL, NKG2D and any combination thereof.

In a second aspect, the present disclosure further provides a nucleic acid comprising a sequence encoding the chimeric antigen receptor of the present disclosure, a vector comprising the nucleic acid, and an immune cell comprising the nucleic acid or the vector.

In an embodiment, the present disclosure provides a nucleic acid comprising a sequence encoding the chimeric antigen receptor of the present disclosure. Preferably, the nucleic acid is DNA or RNA.

In an embodiment, the present disclosure provides a vector comprising the nucleic acid described above. Specifically, the vector is selected from the group consisting of a linear nucleic acid molecule, a plasmid, a retrovirus, a lentivirus, an adenovirus, a vaccinia virus, a Rous sarcoma virus (RSV), a polyoma virus and an adeno-associated virus (AAV), a bacteriophage, a phagemid, a cosmid or an artificial chromosome. In some embodiments, the vector further comprises an origin of autonomous replication in immune cells, a selectable marker, a restriction enzyme cleavage site, a promoter, a polyA tail (polyA), a 3'UTR, a 5'UTR, an enhancer, a terminator, an insulator, an operon, a selectable marker, a reporter gene, a targeting sequence, and/or a protein purification tag. In a specific embodiment, the vector is an in vitro transcription vector.

In an embodiment, the present disclosure provides an engineered immune cell comprising the nucleic acid or vector of the present disclosure, which expresses the chimeric antigen receptor of the present disclosure. In a specific embodiment, the immune cell is selected from the group consisting of a T cell, a macrophage, a dendritic cell, a monocyte, an NK cell, and an NKT cell. Preferably, the T cell is a CD4+/CD8+ double-positive T cell, a CD4+ helper T cell, a CD8+ T cell, a tumor infiltrating cell, a memory T cell, a naive T cell, a CD4-CD8-T cell, a regulatory T cell, a γδ-T cell, or an αβ-T cell.

In an embodiment, the engineered immune cell comprises suppressed or silenced expression of endogenous CD7 and at least one TCR/CD3 gene. Preferably, the immune cell further comprises suppressed or silenced expression of at least one MHC class II related gene.

In an embodiment, the TCR/CD3 gene is selected from the group consisting of TRAC, TRBC, CD3γ, CD3δ, CD3ε, CD3ζ, and a combination thereof. The MHC-II related gene is selected from the group consisting of HLA-DPA, HLA-DQ, HLA - DRA, RFX5, RFXAP, RFXANK, CIITA, and a combination thereof. In a specific embodiment, the engineered immune cell comprises suppressed or silenced expression of endogenous CD7, at least one TCR/CD3 gene selected from the group consisting of TRAC and TRBC, and at least one MHC class II related gene selected from the group consisting of RFX5, RFXAP, RFXANK and CIITA.

In a third aspect, the present disclosure provides a pharmaceutical composition comprising the chimeric antigen receptor as defined above or encoding nucleic acid thereof, the vector or the engineered immune cells comprising them and one or more pharmaceutically acceptable excipients.

In a fourth aspect, the present disclosure further provides a method for treating a subject with a disease associated with CD7 expression, comprising administering to the subject an effective amount of the engineered immune cells or the pharmaceutical composition according to the present disclosure. Therefore, the present disclosure further covers the use of the engineered immune cells and composition comprising the same in the preparation of a medicine for treating a disease associated with CD7 expression.

In an embodiment, the disease associated with CD7 expression is preferably selected from the group consisting of: acute lymphoblastic leukemia (ALL, such as T-ALL, NK-ALL), acute myeloid leukemia (AML), chronic myelocytic leukemia, chronic lymphocytic leukemia, chronic myelogenous leukemia, T-lymphoblastic lymphoma (T-LBL), non-Hodgkin's lymphoma, peripheral T-cell lymphoma (PTCL), extranodal NK/T-cell lymphoma, γδT-cell lymphoma, and early T-cell precursor lymphoblastic leukemia (ETP-ALL) .

### Detailed Description of Embodiments

Unless otherwise specified, all scientific and technical terms used herein have the same meaning as commonly understood by one of ordinary skill in the art to which this disclosure belongs.

### Chimeric antigen receptors

As used herein, the term "chimeric antigen receptor" or "CAR" refers to an artificially constructed hybrid polypeptide whose basic structure includes an antigen-binding region (such as the antigen-binding portion of an antibody), a transmembrane domain, at least one co-stimulatory domain and a primary signaling domain. CARs are able to redirect the specificity and reactivity of T cells and other immune cells to a selected target in a non-MHC-restricted manner through the antigen-binding properties of monoclonal antibodies. Non-MHC-restricted antigen recognition confers on CAR-expressing T cells the ability to recognize antigens independently of antigen processing, thus bypassing major mechanisms of tumor escape. Furthermore, CARs advantageously do not dimerize with the alpha and beta chains of the endogenous T cell receptor (TCR) when expressed on T cells.

In an embodiment, the novel chimeric antigen receptor provided by the present disclosure comprises an antigen binding region, a transmembrane domain and an intracellular signaling region, wherein the antigen binding region comprises an antibody specifically targeting CD7, and the intracellular signaling region consists of at least one co-stimulatory domain, a primary signaling domain, and a γc chain or intracellular region thereof. In other words, the intracellular region of the chimeric antigen receptor of the present disclosure does not comprise other structures with signal transduction functions except the co-stimulatory domain, the primary signaling domain, and the γc chain or intracellular region thereof. Therefore, compared with the intracellular region of the traditional CAR structure (including the primary signaling domain, or the primary signaling domain and the co-stimulatory domain), the intracellular region of the novel chimeric antigen receptor of the present disclosure further comprises the γc chain or intracellular region thereof. In one embodiment, the co-stimulatory domain, primary signaling domain, and γc chain or intracellular region thereof comprised in the intracellular signaling region are arranged in the order from near to far from the cell membrane. That is, the co-stimulatory domain is the closest to the cell membrane, while the γc chain or intracellular region thereof is the farthest from the cell membrane. As used herein, "antigen-binding region" refers to any structure or functional variant thereof that can bind to an antigen. The antigen binding region may be an antibody or an antigen binding portion thereof. As used herein, the term "antibody" has the broadest meaning understood by those skilled in the art and includes monoclonal antibodies (including whole antibodies), polyclonal antibodies, multivalent antibodies, multispecific antibodies (e.g., bispecific antibodies), and antibody fragments or synthetic polypeptides carrying one or more CDR sequences capable of exhibiting the desired biological activity. Antibody of the present disclosure further comprises a recombinant antibody, a human antibody, a humanized antibody, a murine antibody, a chimeric antibody, or an antigen-binding portion thereof. The term "antibody fragment" or "antigen-binding portion" refers to a portion of an intact antibody, and typically comprises the antigen-binding site of the intact antibody and thus retains the ability to bind to an antigen. Examples of antibody fragments in the present disclosure include, but are not limited to: Fab, Fab', F(ab')₂, Fd fragment, Fd', Fv fragment, scFv, disulfide-linked Fv (sdFv), antibody heavy chain variable region (VH) or light chain variable region (VL), linear antibody, "diabody" with two antigen binding sites, single domain antibody, nanobody, a natural ligand of an antigen or a functional fragment thereof. Accordingly, an "antibody" of the present disclosure encompasses an antibody fragment or an antigen-binding portion of an antibody as defined above.

In an embodiment, the antibody of the present disclosure is selected from the group consisting of IgG, Fab, Fab', F(ab')₂, Fd, Fd', Fv, scFv, sdFv, linear antibody, single domain antibody, nanobody and diabody, preferably selected from the group consisting of Fab, scFv, single domain antibody and nanobody, most preferably scFv. In the present disclosure, antibodies may be monovalent or bivalent, and may be monospecific, bispecific or multispecific antibodies.

"Fab" refers to either of two identical fragments of an immunoglobulin molecule produced after cleavage by papain, consisting of the complete light chain and the N-terminal portion of the heavy chain linked by a disulfide bond, wherein the N-terminal portion of the heavy chain includes the heavy chain variable region and CH1. Compared with intact IgG, Fab has no Fc fragment, has higher fluidity and tissue penetration ability, and can monovalently bind antigen without mediating antibody effect.

"Single-chain antibody" and "scFv" refer to an antibody formed by linking the heavy chain variable region (VH) and the light chain variable region (VL) of an antibody through a linker. The optimal length and/or amino acid composition of the linker can be selected. The length of the linker may significantly affect the folding and interaction of the variable domain of scFv. In fact, if shorter linkers (e.g., with between 5-10 amino acids) are used, intrachain folding may be prevented. For selection of linker size and composition, see, e.g., Hollinger et al., 1993 Proc Natl Acad. Sci. U.S.A. 90:6444-6448; U.S. Patent Application Publication Nos. 2005/0100543, 2005/0175606, 2007/0014794 and PCT Publication Nos. WO2006/020258 and WO2007/024715, the entire contents of which are incorporated herein by reference. Commonly used linkers are, for example, GSTSGSGKPGSGEGSTKG (SEQ ID NO: 66), GGGGSGGGGSGGGGS (SEQ ID NO: 67). A scFv may comprise VH and VL linked in any order, e.g. VH-linker-VL or VL-linker-VH.

"Single domain antibody" or "sdAb" refers to an antibody naturally devoid of light chains, which comprises only a heavy chain variable region (VHH) and two conventional CH2 and CH3 regions, also known as "heavy chain antibody".

"Nanobody" or "Nb" refers to a VHH structure cloned and expressed separately, which has the same structural stability and antigen-binding activity as the original heavy chain antibody, and is currently the smallest unit known to bind the target antigen.

The term "functional variant" or "functional fragment" refers to a variant comprising essentially the amino acid sequence of a parent but containing at least one amino acid modification (i.e. substitution, deletion or insertion) compared to the parent amino acid sequence, provided that the variant retains the biological activity of the parent amino acid sequence. In one embodiment, the amino acid modification is preferably a conservative modification.

As used herein, the term "conservative modification" refers to an amino acid modification that does not significantly affect or alter the binding characteristics of an antibody or antibody fragment comprising the amino acid sequence. These conservative modifications include amino acid substitutions, additions and deletions. Modifications can be introduced into the antibodies of the present disclosure by standard techniques known in the art, such as site-directed mutagenesis and PCR-mediated mutagenesis. A conservative substitution of amino acid is one in which an amino acid residue is replaced by an amino acid residue with a similar side chain. Families of amino acid residues with similar side chains have been defined in the art and include those with basic side chain (e.g., lysine, arginine, histidine), acidic side chain (e.g., aspartic acid, glutamic acid), uncharged polar side chain (e.g., glycine, asparagine, glutamine, serine, threonine, tyrosine, cysteine), non-polar side chain (e.g., alanine, valine, leucine, isoleucine, proline, phenylalanine, methionine, tryptophan), beta-branched side chain (e.g., threonine, valine, isoleucine) and aromatic side chain (e.g., tyrosine, phenylalanine, tryptophan, histidine). Conservative modifications can be selected, for example, on the basis of similarity in polarity, charge, solubility, hydrophobicity, hydrophilicity, and/or the amphipathic nature of the residues involved.

Thus, a "functional variant" or "functional fragment" has at least 75%, preferably at least 76%, at least 77%, at least 78%, at least 79%, at least 80%, at least 81 %, at least 82%, at least 83%, at least 84%, at least 85%, at least 86%, at least 87%, at least 88%, at least 89%, at least 90%, at least 91%, at least 92%, at least 93%, at least 94%, at least 95%, at least 96%, at least 97%, at least 98%, or 99% sequence identity to the parent amino acid sequence and retains the biological activity of the parent amino acid, such as binding activity.

As used herein, the term "sequence identity" means the degree to which two (nucleotide or amino acid) sequences in alignment have the same residue at the same position, and is usually expressed as a percentage. Preferably, identity is determined over the entire length of the sequences being compared. Therefore, two copies of the exact same sequence have 100% identity. Those skilled in the art will recognize that several algorithms can be used to determine sequence identity using standard parameters, such as Blast (Altschul et al. (1997) Nucleic Acids Res. 25:3389-3402), Blast2 (Altschul et al. (1990) J. Mol. Biol. 215: 403-410), Smith-Waterman (Smith et al. (1981) J. Mol. Biol. 147:195-197) and Clustal W.

In an embodiment, the antibody targeting CD7 comprised in the chimeric antigen receptor of the present disclosure comprises CDR-L1, CDR-L2, and CDR-L3 as set forth in SEQ ID NO: 1, 2, and 3, respectively, and CDR-H1, CDR-H2 and CDR-H3 as set forth in SEQ ID NO: 4, 5 and 6, respectively. Preferably, the antibody targeting CD7 of the present disclosure comprises a light chain variable region having at least 70%, preferably at least 80%, more preferably at least 90%, at least 95%, at least 97%, at least 98%, at least 99% or 100% sequence identity to an amino acid sequence selected from the group consisting of SEQ ID NOs: 7, 10, 13, 16 and 19, and a heavy chain variable region having at least 70%, preferably at least 80%, more preferably at least 90%, at least 95%, at least 97%, at least 98%, at least 99% or 100% sequence identity to an amino acid sequence selected from the group consisting of SEQ ID NOs: 8, 11, 14, 17 and 20. More preferably, the chimeric antigen receptor of the present disclosure comprises an anti-CD7 antibody with an amino acid sequence set forth in SEQ ID NO: 9, 12, 15, 18 or 21.

In an embodiment, in addition to the antibody targeting CD7, the antigen binding region of the chimeric antigen receptor further comprises an antibody targeting a second antigen, wherein the second antigen is selected from the group consisting of: TSHR, CD19, CD123, CD22, BAFF-R, CD30, CD171, CS-1, CLL-1, CD33, EGFRvIII, GD2, GD3, BCMA, GPRC5D, Tn Ag, PSMA, ROR1, FLT3, FAP, TAG72, CD38 , CD44v6, CEA, EPCAM, B7H3, KIT, IL-13Ra2, mesothelin, IL-I IRa, PSCA, PRSS21, VEGFR2, LewisY, CD24, PDGFR-β, SSEA-4, CD20, AFP, Folate receptor α, ERBB2 (Her2/neu), MUC1, EGFR, CS1, CD138, NCAM, Claudin18.2, Prostase, PAP, ELF2M, Ephrin B2, IGF-I receptor, CAlX, LMP2, gploo, bcr-abI, tyrosinase, EphA2, Fucosyl GM1, sLe, GM3, TGS5, HMWMAA, o-acetyl-GD2, Folate receptor β, TEM1/CD248, TEM7R, CLDN6, GPRC5D, CXORF61, CD97, CD 179a, ALK, polysialic acid, PLAC1, GloboH, NY-BR-1, UPK2, HAVCR1, ADRB3, PANX3, GPR20, LY6K, OR51E2, TARP, WT1, NY-ESO-1, LAGE-1a, MAGE-A1, legumain, HPV E6, E7, MAGE A1, ETV6-AML, sperm protein 17, XAGE1, Tie 2, MAD-CT-1, MAD-CT-2, Fos associated antigen 1, p53, p53 mutant, prostate specific protein, survivin and telomerase, PCTA-1/Galectin 8, MelanA/MART1, Ras mutant, hTERT, sarcoma translocation breakpoint, ML-IAP, ERG (TMPRSS2 ETS fusion gene), NA17, PAX3, androgen receptor, Cyclin B1, MYCN, RhoC, TRP-2, CYP1B 1, BORIS, SART3, PAX5, OY-TES 1, LCK, AKAP-4, SSX2, RAGE-1, human telomerase reverse transcriptase, RU1, RU2, intestinal tract carboxylesterase, mut hsp70-2, CD79a, CD79b, CD72, LAIR1, FCAR, LILRA2, CD300LF, CLEC12A, BST2, EMR2, LY75, GPC3, FCRL5, IGLL1, PD1, PDL1, PDL2, TGFβ, APRIL, NKG2D and any combination thereof. Preferably, the second antigen is selected from the group consisting of CD19, CD20, CD22, CD30, CD33, CD38, CD123, CD138, CD171, MUC1, AFP, Folate receptor α, CEA, PSCA, PSMA, Her2, EGFR, IL13Ra2, GD2 , NKG2D, EGFRvIII, CS1, BCMA, mesothelin, and any combination thereof. Antibodies known in the art targeting the above tumor antigens can be used in the present disclosure.

In a preferred embodiment, the antibody targeting the second antigen is an antibody targeting CD19, comprising:
(1) CDR-L1 as set forth in SEQ ID NO: 44, CDR-L2 as set forth in SEQ ID NO: 45, CDR-L3 as set forth in SEQ ID NO: 46, CDR-H1 as set forth in SEQ ID NO: 47, CDR-H2 as set forth in SEQ ID NO: 48 and CDR-H3 as set forth in SEQ ID NO: 49; or
(2) CDR-L1 as set forth in SEQ ID NO: 50, CDR-L2 as set forth in SEQ ID NO: 51, CDR-L3 as set forth in SEQ ID NO: 52, CDR-H1 as set forth in SEQ ID NO: 53, CDR-H2 as set forth in SEQ ID NO: 54 and CDR-H3 as set forth in SEQ ID NO: 55.

In a preferred embodiment, the antibody targeting the second antigen is an antibody targeting CD19, which comprises a light chain variable region having at least 70%, preferably at least 80%, more preferably at least 90%, at least 95%, at least 97%, at least 99% or 100% sequence identity to the amino acid sequence set forth in SEQ ID NO: 56 or 59, and a heavy chain variable region having at least 70%, preferably at least 80%, more preferably at least 90%, at least 95%, at least 97%, at least 99% or 100% sequence identity to the amino acid sequence set forth in SEQ ID NOs: 57 or 60. More preferably, the chimeric antigen receptor of the present disclosure comprises a CD7-targeting antibody and a CD19-targeting antibody, and the amino acid sequence of the CD19-targeting antibody is as set forth in SEQ ID NO: 58 or 61.

As used herein, the term "transmembrane domain" refers to a polypeptide structure that enables expression of a chimeric antigen receptor on the surface of an immune cell (e.g., a lymphocyte, an NK cell, or an NKT cell), and guides a cellular response of the immune cell against the target cell. The transmembrane domain may be natural or synthetic, and also may be derived from any membrane-bound protein or transmembrane protein. The transmembrane domain is capable of signaling when the chimeric receptor polypeptide binds to the target antigen. The transmembrane domains particularly suitable for use in the present disclosure may be derived from, for example, TCRα chain, TCRβ chain, TCRγ chain, TCRδ chain, CD3ζ subunit, CD3ε subunit, CD3γ subunit, CD3δ subunit, CD45, CD4, CD5, CD8α, CD9, CD16, CD22, CD33, CD28, CD37, CD64, CD80, CD86, CD134, CD137, CD154, and functional fragments thereof. Alternatively, the transmembrane domain may be synthesized and may mainly comprise hydrophobic residues such as leucine and valine. Preferably, the transmembrane domain is derived from human CD8α chain, and has at least 70%, preferably at least 80%, more preferably at least 90%, at least 95%, at least 97% or at least 99% or 100% sequence identity to the amino acid sequence as set forth in SEQ ID NO: 22, or the encoding sequence thereof has at least 70%, preferably at least 80%, more preferably at least 90%, at least 95%, at least 97% or at least 99% or 100% sequence identity to the nucleotide sequence as set forth in SEQ ID NO: 23.

In an embodiment, the chimeric antigen receptors of the present disclosure may further comprise a hinge region located between the antigen binding region and the transmembrane domain. As used herein, the term "hinge region" generally refers to any oligopeptide or polypeptide that functions to link a transmembrane domain to an antigen binding region. Specifically, the hinge region serves to provide greater flexibility and accessibility to the antigen binding region. The hinge region may comprise up to 300 amino acids, preferably 10 to 100 amino acids and most preferably 25 to 50 amino acids. The hinge region may be completely or partially derived from a natural molecule, for example, completely or partially from the extracellular region of CD8, CD4 or CD28, or completely or partially from an antibody constant region. Alternatively, the hinge region may be a synthetic sequence corresponding to a naturally occurring hinge sequence, or may be a completely synthetic hinge sequence. In a preferred embodiment, the hinge region comprises a hinge region portion of CD8α chain, CD28, CD4, FcγRIIIα receptor, IgG4 or IgG1, more preferably a hinge from CD8α, CD28 or IgG4, and has at least 70%, preferably at least 80%, more preferably at least 90%, at least, at least 97% or at least 99% or 100% sequence identity to the amino acid sequence as set forth in SEQ ID NO: 38, 40 or 42, or the encoding sequence thereof has at least 70%, preferably at least 80%, more preferably at least 90%, at least 95%, at least 97% or at least 99% or 100% sequence identity to the nucleotide sequence as set forth in SEQ ID NO: 39, 41 or 43.

As used herein, the term "primary signaling domain" refers to a protein portion that transduces an effector function signal and guides a cell to perform a specified function. The primary signaling domain is responsible for intracellular primary signaling following antigen binding at the antigen binding region, resulting in activation of immune cells and immune responses. In other words, the primary signaling domain is responsible for activating at least one of the normal effector functions of the immune cell in which the CAR is expressed. For example, the effector function of a T cell may be cytolytic activity or helper activity, including secretion of cytokines.

In an embodiment, the primary signaling domain comprised in the chimeric antigen receptor of the present disclosure may be the cytoplasmic sequences of a T cell receptor and a co-receptor, upon binding of antigen receptor, which act together to initiate primary signaling, as well as any derivative or variant of these sequences and any synthetic sequence having the same or similar function. The primary signaling domain may comprise many immunoreceptor tyrosine-based activation motifs (ITAM). Non-limiting examples of the primary signaling domain of the present disclosure include, but are not limited to, those derived from FcRγ, FcRβ, CD3γ, CD3δ, CD3ε, CD3ζ, CD22, CD79a, CD79b, and CD66d. In a preferred embodiment, the primary signaling domain of the CAR of the present disclosure may comprise a CD3ζ signaling domain, which has at least 70%, preferably at least 80%, more preferably at least 90%, at least 95%, at least 97% or at least 99% or 100% sequence identity to the amino acid sequence as set forth in SEQ ID NO: 30 or 32, or the encoding sequence thereof has at least 70%, preferably at least 80%, more preferably at least 90%, at least 95%, at least 97% or at least 99% or 100% sequence identity to the nucleotide sequence as set forth in SEQ ID NO: 31 or 33.

In an embodiment, the intracellular signaling region of the chimeric antigen receptor of the present disclosure may comprise one or more co-stimulatory domains. The co-stimulatory domain may be an intracellular functional signaling domain from a co-stimulatory molecule, which comprises the entire intracellular portion of the co-stimulatory molecule, or a functional fragment thereof. A "co-stimulatory molecule" refers to a cognate binding partner that specifically binds to a co-stimulatory ligand on a T cell, thereby mediating a co-stimulatory response (e.g., proliferation) of the T cell. Co-stimulatory molecules include, but are not limited to, MHC class 1 molecules, BTLA, and Toll ligand receptors. Non-limiting examples of co-stimulatory domains of the present disclosure include, but are not limited to, co-stimulatory signaling domains derived from the following proteins: TLR1, TLR2, TLR3, TLR4, TLR5, TLR6, TLR7, TLR8, TLR9, TLR10, CARD11, CD2, CD7, CD8, CD18 (LFA-1), CD27, CD28, CD30, CD40, CD54 (ICAM), CD83, CD134 (OX40), CD137 (4-1BB), CD270 (HVEM), CD272 (BTLA), CD276 (B7-H3), CD278 (ICOS), CD357 (GITR), DAP10, LAT, NKG2C, SLP76, PD-1, LIGHT, TRIM, CD94, LTB, and ZAP70.

In a preferred embodiment, the co-stimulatory domain comprises one or more intracellular regions of a protein selected from the group consisting of DAP10, DAP12, CD27, CD28, CD134, 4-1BB or CD278. For example, in an embodiment, the co-stimulatory domain comprises the intracellular region of 4-1BB. In an embodiment, the co-stimulatory domain comprises the intracellular region of CD28. In an embodiment, the co-stimulatory domain comprises the intracellular region of 4-1BB and the intracellular region of CD28.

In an embodiment, the intracellular region of 4-1BB has at least 70%, preferably at least 80%, more preferably at least 90%, at least 95%, at least 97% or at least 99% or 100% sequence identity to the amino acid sequence as set forth in SEQ ID NO: 28, or the coding sequence thereof has at least 70%, preferably at least 80%, more preferably at least 90%, at least 95%, at least 97% or at least 99% or 100% sequence identity to the nucleotide sequence as set forth in SEQ ID NO: 29. In an embodiment, the intracellular region of CD28 has at least 70%, preferably at least 80%, more preferably at least 90%, at least 95%, at least 97% or at least 99% or 100% sequence identity to the amino acid sequence as set forth in SEQ ID NO: 26, or the coding sequence thereof has at least 70%, preferably at least 80%, more preferably at least 90%, at least 95%, at least 97% or at least 99% or 100% sequence identity to the nucleotide sequence as set forth in SEQ ID NO: 27.

In an embodiment, in addition to the co-stimulatory domain and the primary signaling domain for signal transduction, the chimeric antigen receptor of the present disclosure further comprises a γc chain or intracellular region thereof to enhance signal transduction. In this embodiment, the intracellular region (i.e., the structure for signal transduction) of the chimeric antigen receptor of the present disclosure is composed of three signaling structures: co-stimulatory domain, primary signaling domain, and γc chain or intracellular region thereof. This means that the chimeric antigen receptors of the present disclosure do not contain a fourth signaling structure, such as the signaling region of other cytokines, such as the intracellular region of IL-2Ra, IL2Ra, IL2Rb, IL4Ra, IL7Ra, IL9Ra, IL 15Ra, IL21 Ra, etc.

As used herein, the term "γc chain" refers to the γ chain shared by the receptors for the cytokines IL-2, IL-4, IL-7, IL-9, IL-15, IL-21. The γc chain was initially identified in the IL-2 receptor, and later found to be involved in the composition of receptors of IL-4, IL-7, IL-9, IL-15, IL-21, etc, so it is also called the universal γ chain. For example, there are three forms of the IL-2 receptor, formed by different combinations of the alpha chain (also known as IL-2Rα), the beta chain (also known as IL-2Rβ), and the gamma chain (also known as IL-2Rγ or IL-2Rg), in which the IL-2 receptor comprising all three chains had the highest affinity for the IL-2 cytokine compared to other combinations. The three chains of the IL-2 receptor are anchored on the cell membrane and transmit biochemical signals into the cell by binding to IL-2. Among these γ chain-dependent cytokines, cytokine receptor-specific components, such as IL-2Rβ, IL-4Rα, IL-7Rα, IL-9Ra, IL-21R, etc., are responsible for binding to JAK1, while the γc chain for binding to JAK3. When these cytokine receptors bind to cytokines, three major signaling pathways are activated, including MAP kinase, PI3 kinase, and JAK-STAT pathway, which in turn regulate the survival and proliferation of T cells and NK cells.

The γc chain is a glycoprotein with a molecular weight of 64kD, consisting of 347 amino acids, including an extracellular region of 232 amino acids, a transmembrane region of 29 amino acids and an intracellular region of 86 amino acids. The intracellular region comprises the Src homology region, which is very important for promoting the growth of cells and the expression of c-myc, c-fos, c-jun and other genes mediated by IL-2. In an embodiment, the γc chain that may be used in the present disclosure has at least 70%, preferably at least 80%, more preferably at least 90%, at least 95%, at least 97% or at least 99% or 100% sequence identity to the amino acid sequence as set forth in SEQ ID NO: 62, or the encoding sequence thereof has at least 70%, preferably at least 80%, more preferably at least 90%, at least 95%, at least 97% or at least 99% or 100% sequence identity to the nucleotide sequence as set forth in SEQ ID NO: 63. Preferably, the intracellular region of the γc chain that may be used in the present disclosure has at least 70%, preferably at least 80%, more preferably at least 90%, at least 95%, at least 97% or at least 99% or 100% sequence identity to the amino acid sequence as set forth in SEQ ID NO: 64, or the encoding sequence thereof has at least 70%, preferably at least 80%, more preferably at least 90%, at least 95%, at least 97% or at least 99% or 100% sequence identity to the nucleotide sequence as set forth in SEQ ID NO: 65. Preferably, the γc chain of the present disclosure is set forth in SEQ ID NO: 63, and the intracellular region thereof is set forth in SEQ ID NO: 65. In an embodiment, the CAR of the present disclosure may further comprise a signal peptide such that when it is expressed in a cell such as a T cell, the nascent protein is directed to the endoplasmic reticulum and subsequently to the cell surface. The core of the signal peptide may comprise a long hydrophobic amino acid segment, which has a tendency to form a single α-helix. At the end of the signal peptide, there is usually an amino acid segment capable of being recognized and cleaved by signal peptidase. The signal peptidase may cleave during or after translocation, so as to generate free signal peptide and mature protein. Then, the free signal peptide is digested by a specific protease. Signal peptides that may be used in the present disclosure are well known to those skilled in the art, for example, signal peptides derived from CD8α, IgG1, GM-CSFRα, and the like.

In an embodiment, the CAR of the present disclosure may further comprise a switch structure to regulate the expression time of the CAR. For example, the switch structure may be in the form of a dimerization domain, which undergoes a conformational change after binding to its corresponding ligand, exposing the extracellular antigen-binding region, allowing it to bind to the targeted antigen, thereby activating the signal transduction pathway. Alternatively, switch structures may also be used to link the antigen-binding domain and the signaling domain separately, and only when the switch structures are coupled with each other (for example, in the presence of an inducing compound), the antigen-binding domain and the signaling domain can be linked together through dimerization, thereby activating the signaling pathway. The switch structure may also be in the form of a masking peptide. The masking peptide can mask the extracellular antigen-binding region and prevent it from binding to the targeted antigen. When the masking peptide is cleaved by, for example, protease, the extracellular antigen-binding region can be exposed, making it a "normal" CAR structure. Various switch configurations known to those skilled in the art can be used in the present disclosure.

In an embodiment, the CAR of the present disclosure may further comprises a suicide gene, which express a cell death signal that can be induced by an exogenous substance, so as to eliminate CAR cells when needed (for example, when severe toxic side effects occur). For example, suicide genes may be in the form of inserted epitopes, such as CD20 epitopes, RQR8, etc., and when necessary, CAR cells can be eliminated by adding antibodies or reagents targeting these epitopes. The suicide gene may also be herpes simplex virus thymidine kinase (HSV-TK), which causes cell death induced by ganciclovir treatment. The suicide gene may also be iCaspase-9, and the dimerization of iCaspase-9 can be induced by chemically inducing drugs such as AP1903 and AP20187, thereby activating the downstream Caspase3 molecule and leading to cell apoptosis. Various suicide genes known to those skilled in the art may be used in the present disclosure.

### Nucleic acids

The present disclosure further provides a nucleic acid comprising a sequence encoding the chimeric antigen receptor of the present disclosure.

As used herein, the term "nucleic acid" includes sequences of ribonucleotides and deoxyribonucleotides, such as modified or unmodified RNA or DNA, each in single- and/or double-stranded form, linear or circular , or mixtures thereof (including hybrid molecules). Thus, nucleic acids according to the present disclosure include DNA (such as dsDNA, ssDNA, cDNA), RNA (such as dsRNA, ssRNA, mRNA, ivtRNA), combinations or derivatives thereof (such as PNA). Preferably, the nucleic acid is DNA or RNA, more preferably mRNA.

Nucleic acids may comprise conventional phosphodiester bonds or unconventional bonds such as amide bonds such as those found in peptide nucleic acids (PNAs). Nucleic acids of the present disclosure may further comprises one or more modified bases for example, tritylated bases and unusual bases such as inosine. Other modifications are also contemplated, including chemical, enzymatic or metabolic modifications, so long as the multi-chain CAR of the present disclosure can be expressed from the polynucleotide. The nucleic acid may be provided in isolated form. In an embodiment, the nucleic acid may further comprise regulatory sequences, such as transcriptional control elements (including promoters, enhancers, operators, repressors, and transcriptional termination signals), ribosome binding sites, introns, and the like.

The nucleic acid sequences of the present disclosure may be codon-optimized for optimal expression in desired host cells (e.g., immune cells); or for expression in bacteria, yeast or insect cells. Codon optimization refers to the replacement of codons in the target sequence that are generally rare in highly expressed genes of a given species with codons that are generally common in highly expressed genes of this species, while the codons before and after the replacement code for the same amino acid. Therefore, the choice of optimal codons depends on the codon usage bias of the host genome.

### Vectors

The present disclosure further provides a vector comprising one or more nucleic acids according to the present disclosure.

As used herein, the term "vector" is an intermediary nucleic acid molecule used to transfer (exogenous) genetic material into a host cell, and in the host cell the nucleic acid molecule can be, for example, replicated and/or expressed.

The vector generally includes targeting vectors and expression vectors. The "targeting vector" is a medium that delivers an isolated nucleic acid to the interior of a cell by, for example, homologous recombination or by using a hybridization recombinase specifically targeting a sequence at a site. The "expression vector" is a vector used for transcription of heterologous nucleic acid sequences (for example, those sequences encoding the chimeric antigen receptor polypeptides of the present disclosure) in suitable host cells and the translation of their mRNAs. Suitable vectors that can be used in the present disclosure are known in the art, and many are commercially available. In an embodiment, the vector of the present disclosure includes, but is not limited to, linear nucleic acid molecule (such as DNA or RNA), plasmid, virus (e.g., retrovirus, lentivirus, adenovirus, vaccinia virus, Rous sarcoma virus (RSV), polyoma virus, and adeno-associated virus (AAV), etc.), bacteriophage, phagemid, cosmid, and artificial chromosome (including BAC and YAC). The vector itself is usually a sequence of nucleotides, and usually is a DNA sequence comprising an insert (transgene) and a larger sequence as "backbone" of the vector. Engineered vector typically also comprises an origin of autonomous replication in the host cell (if stable expression of polynucleotide is desired), a selectable marker, and a restriction enzyme cleavage site (e.g., a multiple cloning site, MCS). The vectors may additionally comprise elements such as a promoter, a poly-A tail (polyA), 3' UTR, an enhancer, a terminator, an insulator, an operon, a selectable marker, a reporter gene, a targeting sequence, and/or a protein purification tag. In a specific embodiment, the vector is an in vitro transcription vector.

### Engineered immune cells

The present disclosure provides an engineered immune cell comprising the chimeric antigen receptor or the encoding nucleic acid thereof.

As used herein, the term "immune cell" refers to any cell of the immune system that has one or more effector functions (e.g., cytotoxic cell killing activity, secretion of cytokines, induction of ADCC and/or CDC). For example, the immune cell may be a T cell, a macrophage, a dendritic cell, a monocyte, an NK cell, and/or an NKT cell. In an embodiment, the immune cell is derived from a stem cell, such as an adult stem cell, an embryonic stem cell, a cord blood stem cell, a progenitor cell, a bone marrow stem cell, an induced pluripotent stem cell, a totipotent stem cell, or a hematopoietic stem cell, and so on. Preferably, the immune cell is a T cell. The T cell may be any T cell, such as in vitro cultured T cell, for example, primary T cell, or T cell from in vitro cultured T cell line, e.g., Jurkat, SupT1, etc., or T cell obtained from a subject. Examples of subject include humans, dogs, cats, mice, rats, and transgenic species thereof. The T cell may be obtained from a variety of sources, including peripheral blood monocytes, bone marrow, lymph node tissue, umbilical blood, thymus tissue, tissue from sites of infection, ascites, pleural effusion, spleen tissue, and tumors. The T cell also may be concentrated or purified. The T cell may be any type of T cell and may be at any stage of development including, but not limited to, CD4+/CD8+ double positive T cell, CD4+ helper T cell (e.g., Th1 and Th2 cells), CD8+ T cell (e.g., cytotoxic T cell), tumor infiltrating cell, memory T cell, naive T cell, CD4-CD8-T cell, regulatory T cell, γδ-T cell, αβ-T cell, etc. In a preferred embodiment, the immune cell is a human T cell. The T cell may be isolated from the blood of a subject using a variety of techniques known to those of skill in the art, such as Ficoll. In the present disclosure, the immune cell is engineered to express a chimeric antigen receptor polypeptide.

The nucleic acid sequence encoding the chimeric antigen receptor polypeptide can be introduced into an immune cell using conventional methods known in the art (e.g., by transduction, transfection, transformation), so that the chimeric antigen receptor polypeptide of the present disclosure can be expressed. "Transfection" is a process of introducing a nucleic acid molecule or polynucleotide (including a vector) into a target cell. An example is RNA transfection, i.e., the process of introducing RNA (e.g., in vitro transcribed RNA, ivtRNA) into a host cell. This term is mainly used for a non-viral method in eukaryotic cells. The term "transduction" is generally used to describe virus-mediated transfer of nucleic acid molecules or polynucleotides. Transfection of animal cells typically involves opening transient pores or "holes" in the cell membrane, so as to allow uptake of material. Transfection may be carried out using calcium phosphate, by electroporation, by extrusion of cells, or by mixing cationic lipids with the material so as to produce liposomes which fuse with the cell membrane and deposit their cargo into the interior. Exemplary techniques for transfecting eukaryotic host cells include lipid vesicle-mediated uptake, heat shock-mediated uptake, calcium phosphate-mediated transfection (calcium phosphate/DNA co-precipitation), microinjection, and electroporation. The term "transformation" is used to describe the non-virus transfer of a nucleic acid molecule or polynucleotide (including a vector) to bacteria, and also to non-animal eukaryotic cells (including plant cells). Thus, the transformation is a genetic alteration of bacterial or non-animal eukaryotic cells, which is produced by direct uptake of a cell membrane from its surroundings and subsequent incorporation of exogenous genetic material (nucleic acid molecule). The transformation may be achieved by artificial means. In order for transformation to occur, the cell or bacterium must be in a competent state. For prokaryotic transformation, the techniques may include heat shock-mediated uptake, fusion to bacterial protoplasts of intact cells, microinjection, and electroporation.

In an embodiment, the expression of endogenous CD7 of the engineered immune cells of the present disclosure is inhibited or silenced. In an embodiment, the expression of at least one TCR/CD3 gene of the engineered immune cells of the present disclosure is inhibited or silenced. In an embodiment, the expression of endogenous CD7 and at least one TCR/CD3 gene of the engineered immune cells of the present disclosure is inhibited or silenced.

CD7 is not only expressed in tumor cells, such as T cell acute lymphoblastic leukemia/lymphoma, acute myelocytic leukemia and chronic myelocytic leukemia, but also expressed in most normal T cells and NK cells. Therefore, in order to avoid mutual recognition and killing between CD7-targeting CAR cells, it is necessary to inhibit or silence the expression of the endogenous CD7 gene of CAR cells.

T cell receptor (TCR) is a characteristic mark on the surface of all T cells, which binds to CD3 through a non-covalent bond to form a TCR/CD3 complex, and binds to the specific MHC-antigen peptide complex on the surface of antigen-presenting cells to generate a specific antigen stimulation signal, activate T cells, and play a killing role. Therefore, inhibiting or silencing the expression of the endogenous TCR/CD3 gene in CAR-T cells can avoid its attack on normal cells or tissues in patients, thereby avoiding or reducing the risk of graft-versus-host disease (GvHD). TCR is a heterodimer composed of two different peptide chains, and usually divided into two types: α/β type and γ/δ type, and more than 95% of peripheral T lymphocytes express TCR α/β. The TCR alpha chain is encoded by the TRAC gene, and the beta chain is encoded by the TRBC gene. Each peptide chain of TCR includes a variable region (V region), a constant region (C region), a transmembrane region and a cytoplasmic region, wherein the cytoplasmic region is very short and does not have the ability to transmit antigen stimulation signals. TCR molecules belong to the immunoglobulin superfamily, and its antigen specificity exists in the V region; the V region has three hypervariable regions, CDR1, CDR2, and CDR3, among which CDR3 has the largest variation, which directly determines the antigen-binding specificity of TCR. When TCR recognizes the MHC-antigen peptide complex, CDR1 and CDR2 recognize and bind to MHC molecules, while CDR3 directly binds to the antigen peptide. CD3 includes four subunits: γ, δ, ε, ζ, and usually exists in the form of dimers εγ, εδ, ζζ. These four subunits all comprise the conserved immunoreceptor tyrosine-based activation motif (ITAM), and after the two tyrosine residues are phosphorylated by tyrosine protein kinase, it transmits an activation signal to T cells. Thus, in an embodiment, at least one TCR/CD3 gene is selected from the group consisting of: TRAC, TRBC, CD3γ, CD3δ, CD3ε, CD3ζ.

In an embodiment, the expression of at least one MHC class II related gene of the engineered immune cells of the present disclosure is inhibited or silenced. In an embodiment, the expression of endogenous CD7 and at least one MHC class II related gene of the engineered immune cells of the present disclosure is inhibited or silenced. In an embodiment, the expression of at least one TCR/CD3 gene and at least one MHC class II related gene of the engineered immune cells of the present disclosure is inhibited or silenced. In an embodiment, the expression of endogenous CD7, at least one TCR/CD3 gene and at least one MHC class II related gene of the engineered immune cells of the present disclosure is inhibited or silenced. In this embodiment, MHC class II related genes include MHC class II genes themselves, as well as genes that interact with or regulate the expression of MHC class II genes.

The major histocompatibility complex (MHC), originally characterized as a protein that plays a major role in the transplant response, is expressed on the surface of all higher vertebrates and is called H-2 in mice, called HLA in human cells. There are two main classes of MHC: class I and class II. MHC class I proteins are heterodimers of two proteins: one is the alpha chain of a transmembrane protein encoded by the MHC I gene, and the other is the β2 microglobulin chain of an extracellular protein encoded by a gene not located within the MHC gene cluster. The alpha chain consists of three domains and the foreign peptide binds to two domains α1, α2 at the N-terminus which are also the most variable. MHC class II proteins are also heterodimers, comprising two transmembrane proteins encoded by genes within the MHC complex. Class I MHC/antigen complexes interact with cytotoxic T cells (e.g., CD8+ T cells), while class II MHC presents antigens to helper T cells (e.g., CD4+ T cells). Furthermore, MHC class I proteins tend to be expressed in almost all nucleated cells and platelets (and red blood cells in mice), whereas MHC class II proteins are more selectively expressed. Typically, MHC class II proteins are expressed on B cells, some macrophages and monocytes, activated T cells, Langerhans cells and dendritic cells.

The human HLA class II cluster contains three major loci DP, DQ and DR. Class II molecules are heterodimers consisting of an alpha chain and a beta chain, both anchored in the membrane, where the alpha chain is approximately 33-35 kDa and contains 5 exons. Exon 1 encodes the leader peptide, exons 2 and 3 encode the two extracellular domains, exon 4 encodes the transmembrane domain, and exon 5 encodes the cytoplasmic tail. Thus, in an embodiment, the MHC class II related genes are selected from the group consisting of: HLA-DPA, HLA-DQ and HLA-DRA.

The expression of MHC class II genes also depends on a variety of important positive regulatory proteins, such as RFX complex, CIITA and so on. The RFX complex is composed of three subunits: RFXANK (also known as RFXB), RFX5, and RFX accessory protein (also known as RFXAP). The RFX complex promotes the expression of MHC class II molecules by facilitating the binding of other transcription factors to the promoters of MHC class II molecules and enhancing the specificity of promoter binding. CIITA is a master controller of MHC class II expression. CIITA comprises an N-terminal rich in acidic amino acids, a PST region rich in Pro, Ser, and Thr, a GTP-binding region in the middle, and a C-terminal rich in Leu repeat sequence (LRR), where the N-terminal acidic region and PST region are transcription activation regions. Thus, in an embodiment, the MHC class II related genes are selected from the group consisting of: RFX5, RFXAP, RFXANK, and CIITA.

In an embodiment, the MHC class II related genes are selected from the group consisting of HLA-DPA, HLA-DQ, HLA-DRA, RFX5, RFXAP, RFXANK and CIITA, preferably selected from the group consisting of RFX5, RFXAP, RFXANK and CIITA, more preferably RFX5 or CIITA.

In an embodiment, the endogenous MHC class I genes (e.g., HLA-A, HLA-B, HLA-C, B2M, etc.) in the CAR-T cells are functional. In another embodiment, the expression of endogenous MHC class I genes in CAR-T cells is also suppressed or silenced.

In an embodiment, the engineered immune cell expressing a chimeric antigen receptor targeting CD7 according to the present disclosure comprises suppressed or silenced expression of endogenous CD7, at least one TCR/CD3 gene, and at least one MHC-II related gene, wherein the at least one TCR/CD3 gene is selected from the group consisting of TRAC, TRBC, CD3γ, CD3δ, CD3ε, CD3ζ and a combination thereof; and at least one MHC-II related gene is selected from the group consisting of HLA-DPA, HLA-DQ, HLA -DRA, RFX5, RFXAP, RFXANK, and CIITA. In a preferred embodiment, the engineered immune cell expressing a chimeric antigen receptor targeting CD7 according to the present disclosure comprises suppressed or silenced expression of endogenous CD7, at least one TCR/CD3 gene selected from the group consisting of TRAC and TRBC, and at least one MHC class II gene selected from the group consisting of RFX5, RFXAP, RFXANK and CIITA. More preferably, the engineered immune cell expressing a chimeric antigen receptor targeting CD7 according to the present disclosure comprises suppressed or silenced expression of endogenous CD7, at least one TCR/CD3 gene selected from the group consisting of TRAC and TRBC, and RFX5. More preferably, the engineered immune cell expressing a chimeric antigen receptor targeting CD7 according to the present disclosure comprises suppressed or silenced expression of endogenous CD7, at least one TCR/CD3 gene selected from the group consisting of TRAC and TRBC, and CIITA. In an embodiment, the expression of MHC class I genes such as HLA-A, HLA-B, HLA-C and B2M in the engineered immune cells is functional.

In an embodiment, in addition to CD7, MHC class II related genes and TCR/CD3 genes, the engineered immune cells of the present disclosure may further comprise suppressed or silenced expression of at least one gene selected from the group consisting of: CD52, GR, dCK and immune checkpoint genes such as PD1, LAG3, TIM3, CTLA4, PPP2CA, PPP2CB, PTPN6, PTPN22, PDCD1, HAVCR2, BTLA, CD160, TIGIT, CD96, CRTAM, TNFRSF10B, TNFRSF10A, CASP8, CASP10, CASP3, CASP6, CASP7, FADD, FAS, TGFBRII, TGFRBRI, SMAD2, SMAD3, SMAD4, SMAD10, SKI, SKIL, TGIF1, IL10RA, IL10RB, HMOX2, IL6R, IL6ST, EIF2AK4, CSK, PAG1, SIT, FOXP3, PRDM1, BATF, GUCY1A2, GUCY1A3, GUCY1B2, and GUCY1B3.

Methods for inhibiting gene expression or gene silencing are well known to those skilled in the art, including but not limited to, for example, mediating DNA fragmentation by meganucleases, zinc finger nucleases, TALE nucleases, or Cas enzymes in the CRISPR system, or inactivating genes through technologies such as antisense oligonucleotides, RNAi, shRNA, etc..

In an embodiment, the engineered immune cell of the present disclosure further expresses an immunosuppressive molecule, wherein the immunosuppressive molecule comprises one or more immune cell antigen binding domains, a transmembrane domain and a co-stimulatory domain. When the engineered immune cells of the present disclosure are administered to a subject, the immunosuppressive molecule can inhibit or reduce the immune response of immune cells (such as NK cells, T cells) in the subject to the exogenous engineered immune cells, thereby reducing the risk of HvG. In an embodiment, the immunosuppressive molecule does not comprise a primary signaling domain. The transmembrane domain, co-stimulatory domain and primary signaling domain are defined as above.

In an embodiment, the immune cell antigen binding region is an antibody targeting an immune cell antigen, a ligand of an immune cell antigen, or a combination thereof. Preferably, the antibody is selected from the group consisting of Fab, Fab', F(ab')₂, Fd, Fd', Fv, scFv, sdFv, a linear antibody, a single domain antibody, a nanobody and a diabody. Preferably, the immune cell antigen is selected from the group consisting of NKG2A, NKG2B, NKG2C, NKG2D, NKG2E, NKP46, LIR1, LIR2, LIR3, LIR5, LIR8, PD-1, TIGIT, TIM3, LAG3, KIR, CEACAM1, LAIR1, SIGLEC7, SIGLCE9, KLRG1, CD2, CD3, CD4, CD5, CD8, CD16a, CD16b, CD25, CD27, CD28, CD30, CD38, CD45, CD48, CD50, CD52, CD56, CD57 , CD62L, CD69, CD94, CD96, CD100, CD102, CD122, CD127, CD132, CD137, CD160, CD161, CD178, CD218, CD226, CD244, CD279, CD305, CD337, CS1, and a combination thereof. Preferably, the immune cell antigen is selected from the group consisting of NKG2A, NKG2B, CD94, LIR1, LIR2, LIR3, SIGLEC7, SIGLCE9, KIR, CEACAM1, LAIR1, KLRG1 and a combination thereof. In an embodiment, the ligand of the immune cell antigen is selected from the group consisting of HLA-E, HLA-F, HLA-G, cadherin (such as E-cadherin, N-cadherin, R-cadherin), collagen, OCIL, sialic acid, PD-L1, PD-L2, CD155, CTLA4, CD112, CD113, Gal-9, FGL1 and extracellular region thereof. Preferably, the ligand of the immune cell antigen is selected from the group consisting of HLA-E, HLA-F, HLA-G, E-cadherin, PD-L1, PD-L2 and extracellular region thereof. In one embodiment, the immune cell antigen binding region comprises an anti-NKG2A antibody, an anti-LIR1 antibody, an anti-SIGLEC7 antibody, an anti-SIGLCE9 antibody, an anti-KIR antibody, an anti-KLRG1 antibody, a PD-L1 extracellular region, a PD-L2 extracellular region, an E-cadherin extracellular region, a HLA-E extracellular region, a HLA-G extracellular region or a combination thereof. In an embodiment, the engineered immune cell further comprises a second chimeric antigen receptor targeting a second antigen, the second antigen being defined above. In this embodiment, the second chimeric antigen receptor may or may not comprise a γc chain or intracellular region thereof.

In an embodiment, a plurality of immune cells is provided, each immune cell engineered to express one or more chimeric antigen receptors. For example, in some embodiments, an immune cell is engineered to express a chimeric antigen receptor that binds and/or targets CD7 (e.g., a CAR comprising the anti-CD7 antibody of the present disclosure), and another cell is engineered to express a chimeric antigen receptor that binds and/or targets antigens different from CD7. In an embodiment, immune cells may also express multispecific chimeric antigen receptors that target one or more antigens, including CD7. For example, such a multispecific chimeric antigen receptor may comprise a multispecific antibody targeting CD7, or comprise both the anti-CD7 antibody of the present disclosure and antibodies targeting antigens different from CD7. In such embodiments, the plurality of engineered immune cells may be administered together or separately. In an embodiment, the plurality of immune cells may be in the same composition or in different compositions. Exemplary compositions of cells include those described in the following sections of this application.

### Pharmaceutical composition

The present disclosure further provides a pharmaceutical composition, which comprises the chimeric antigen receptor, the nucleic acid, the vector or the engineered immune cell described in the present disclosure as an active agent, and one or more pharmaceutically acceptable excipients. Therefore, the present disclosure also covers the use of the chimeric antigen receptor, the nucleic acid, the vector or the engineered immune cell in the preparation of a pharmaceutical composition or medicine.

As used herein, the term "pharmaceutically acceptable excipient" refers to a carrier and/or excipient that is pharmacologically and/or physiologically compatible (i.e., capable of triggering a desired therapeutic effect without causing any undesired local or systemic effects) with the subject and active ingredient, and it is well known in the art (see, e.g., Remington's Pharmaceutical Sciences. Edited by Gennaro AR, 19th ed. Pennsylvania: Mack Publishing Company, 1995). Examples of pharmaceutically acceptable excipient include, but are not limited to, filler, binder, disintegrant, coating agent, adsorbent, anti-adherent, glidant, antioxidant, flavoring agent, colorant, sweetener, solvent, co-solvent, buffer agent, chelating agent, surfactant, diluent, wetting agent, preservative, emulsifier, cladding agent, isotonic agent, absorption delaying agent, stabilizer, and tension regulator. It is known to those skilled in the art to select a suitable excipient to prepare the desired pharmaceutical composition of the present disclosure. Exemplary excipients for use in the pharmaceutical composition of the present disclosure include saline, buffered saline, dextrose, and water. Generally, the selection of a suitable excipient depends, in particular, on the active agent used, the disease to be treated, and the desired dosage form of the pharmaceutical composition.

The pharmaceutical composition according to the present disclosure is suitable for multiple routes of administration. Generally, the administration is parenterally accomplished. Parenteral delivery methods comprise topical, intraarterial, intramuscular, subcutaneous, intramedullary, intrathecal, intraventricular, intravenous, intraperitoneal, intrauterine, intravaginal, sublingual, or intranasal administration.

The pharmaceutical composition according to the present disclosure further may be administered in combination with one or more other agents suitable for the treatment and/or prophylaxis of diseases to be treated. Preferred examples of agent suitable for the combination include known anti-cancer medicines such as cisplatin, maytansine derivatives, rachelmycin, calicheamicin, docetaxel, etoposide, gemcitabine, ifosfamide, irinotecan, melphalan, mitoxantrone, sorfimer sodiumphotofrin II, temozolomide, topotecan, trimetreate glucuronate, auristatin E, vincristine and doxorubicin; peptide cytotoxins, such as ricin, diphtheria toxin, pseudomonas exotoxin A, DNase and RNase; radionuclides such as iodine 131, rhenium 186, indium 111, iridium 90, bismuth 210, bismuth 213, actinides 225 and astatine 213; prodrugs such as antibody-directed enzyme prodrugs; immunostimulants, such as IL-2, chemokines such as IL-8, platelet factor 4, melanoma growth stimulating protein, etc.; antibodies or fragments thereof, such as anti-CD3 antibodies or fragments thereof, complement activators, heterologous protein domains, homologous protein domains, viral/bacterial protein domains and viral/bacterial peptides. In addition, the pharmaceutical composition of the present disclosure also can be used in combination with one or more other treatment methods, such as chemotherapy and radiotherapy.

### Therapeutic application

The present disclosure further provides a method for treating a subject with a disease associated with CD7 expression, comprising administering to the subject an effective amount of the immune cells or the pharmaceutical composition according to the present disclosure. Therefore, the present disclosure also covers the use of the engineered immune cells and the pharmaceutical composition in the preparation of medicines for treating diseases associated with CD7 expression.

In an embodiment, an effective amount of the immune cell and/or the pharmaceutical composition of the present disclosure is administered directly to a subject.

In another embodiment, the method of treatment of the present disclosure is ex vivo. Specifically, the method comprises the following steps: (a) providing a sample comprising immune cells; (b) introducing the chimeric antigen receptor of the present disclosure and other exogenous genes (if any) into the immune cells in vitro, and inhibiting or silencing the expression of specific genes in the immune cells (such as endogenous CD7, TCR/CD3 genes and MHC class II related genes), to obtain modified immune cells, and (c) administering the modified immune cells to a subject in need thereof. Preferably, the immune cells provided in step (a) are selected from macrophages, dendritic cells, monocytes, T cells, NK cells and/or NKT cells; and the immune cells can be obtained from a subject's sample (especially a blood sample) by conventional methods known in the art. However, other immune cells capable of expressing the chimeric antigen receptors of the present disclosure and functioning as desired biological effectors as described herein may also be used. Furthermore, immune cells are generally selected to be compatible with the subject's immune system, i.e. preferably the immune cells do not elicit an immunogenic response. For example, "universal recipient cells" are generally compatible lymphocytes functioning as desired biological effectors that can be grown and expanded in vitro. Use of such cells would eliminate the need to obtain and/or provide the subject's own lymphocytes. The ex vivo introduction of step (c) can be carried out by introducing the nucleic acid or vector described herein into the immune cells via electroporation or by infecting the immune cells with a viral vector, which is lentiviral vector, adenoviral vector, adeno-associated viral vector or retroviral vector as dsicribed above. Other conceivable methods include the use of transfection reagents (such as liposomes) or transient RNA transfection.

In one embodiment, the immune cells are autologous or allogeneic cells, preferably T cells, macrophages, dendritic cells, monocytes, NK cells and/or NKT cells, more preferably T cells, NK cells or NKT cells.

As used herein, the term "autologous" refers to any material derived from an individual that will later be reintroduced into that same individual.

As used herein, the term "allogeneic" refers to any material derived from a different animal or a different patient of the same species as the individual into whom the material is introduced. Two or more individuals are considered allogeneic to each other when the genes at one or more loci differ. In some cases, allogeneic material from individuals of the same species may be genetically different enough for antigenic interactions to occur.

As used herein, the term "subject" is a mammal. Mammals can be humans, non-human primates, mice, rats, dogs, cats, horses, or cows, but are not limited to thereto. Mammals other than humans can be advantageously used as subjects representing animal models of cancer. Preferably, the subject is a human.

In an embodiment, diseases associated with CD7 expression include non-solid tumors (such as hematological tumors, e.g., leukemias and lymphomas) and solid tumors. Hematological neoplasms are cancers of the blood or bone marrow, including but not limited to acute leukemias such as acute lymphoblastic leukemia (ALL, e.g. T-ALL, NK-ALL), acute myeloid leukemia (AML), acute myelogenous leukemia and myeloblastic, promyelocytic, myelomonocytic, monocytic, and erythroid leukemia; chronic leukemia, such as chronic myelocytic leukemia, chronic myelogenous leukemia, and chronic lymphocytic leukemia; polycythemia vera, lymphoma , Hodgkin lymphoma, non-Hodgkin lymphoma (indolent and high-grade forms), peripheral T-cell lymphoma (PTCL), angioimmunoblastic T-cell lymphoma (AITL), anaplastic large cell lymphoma (ALCL), extranodal NK/T-cell lymphoma, multiple myeloma, Waldenstrom's macroglobulinemia, myelodysplastic syndrome, hairy cell leukemia, Burkitt lymphoma, diffuse large cell lymphoma, mantle cell lymphoma, T-lymphoblastic lymphoma (T-LBL), γδ T-cell lymphoma, early T-cell precursor lymphoblastic leukemia (ETP-ALL), small lymphocytic lymphoma (SLL) and myeloid dysplasia. Solid tumors are abnormal masses of tissue that usually do not contain cysts or areas of fluid, which can be benign or malignant. The different types of solid tumors are named for the type of cells that form them (such as sarcomas, carcinomas, and lymphomas). Examples of solid tumors include, but are not limited to, fibrosarcoma, myxosarcoma, liposarcoma, mesothelioma, pancreatic cancer, ovarian cancer, carcinoma of the peritoneum, omentum, and mesentery, pharyngeal cancer, prostate cancer, rectal cancer, kidney cancer, skin cancer, small bowel cancer, melanoma, kidney cancer, laryngeal cancer, soft tissue cancer, stomach cancer, testicular cancer, colon cancer, esophagus cancer, cervical cancer, alveolar rhabdomyosarcoma, bladder cancer, bone cancer, brain cancer, breast cancer, anal cancer, eye cancer, intrahepatic cholangiocarcinoma, joint cancer, neck cancer, gallbladder cancer, pleura cancer, nasal cancer, middle ear cancer, oral cancer, vulvar cancer, thyroid cancer and ureter cancer.

In an embodiment, the disease associated with CD7 expression is preferably selected from the group consisting of: acute lymphoblastic leukemia (ALL, such as T-ALL, NK-ALL), acute myeloid leukemia (AML), chronic myelocytic leukemia, chronic lymphocytic leukemia, chronic myelogenous leukemia, T-lymphoblastic lymphoma (T-LBL), non-Hodgkin's lymphoma, peripheral T-cell lymphoma (PTCL), extranodal NK/T-cell lymphoma, γδT-cell lymphoma, and early T-cell precursor lymphoblastic leukemia (ETP-ALL) .

The present disclosure will be described in detail below with reference to the accompanying drawings and examples. It should be noted that those skilled in the art should understand that the drawings and the embodiments of the present disclosure are only for the purpose of illustration, and shall not constitute any limitation to the present disclosure. In the case of no contradiction, the embodiments in the present application and the features in the embodiments can be combined with each other.

### Brief Description of Drawings

Figure 1 shows the expression level of scFv on bbz-dKO T cells and bbzg-dKO T cells.
Figure 2 shows the killing ability of bbz-dKO T cells and bbzg-dKO T cells on target cells.
Figure 3 shows the cytokine release level after co-culture of bbz-dKO T cells and bbzg-dKO T cells with target cells.
Figure 4 shows the expression level of scFv on bbz-tKO T cells and bbzg-tKO T cells.
Figure 5 shows the killing ability of bbz-tKO T cells and bbzg-tKO T cells on target cells.
Figure 6 shows the cytokine release level after co-culture of bbz-tKO T cells and bbzg-tKO T cells with target cells.
Figure 7 shows the killing ability of CAR7X19 T cells on two target cells.
Figure 8 shows the cytokine release level after co-culture of CAR7x19 T cells with two target cells.
Figure 9 shows the inhibitory effect of UNKi-T cells expressing immunosuppressive molecules No.1 (A), No.2-3 (B), and No.4-10 (C) on the killing effect of NK cells. Mock T: T cells with TRAC/B2M/RFX5 triple-gene knockout.
Fig. 10 shows the inhibitory effect of UNKi-T cells expressing immunosuppressive molecules No.11-13 (A, Mock T: B2M knockout T cells) and No.14 (B, Mock T: B2M knockout T cells) on the killing effect of NK cells.
Figure 11 shows the killing ability of bbzg-EA CAR-T cells and bbzg-PA CAR-T cells on target cell Jurkat.
Figure 12 shows the inhibitory effect of bbzg-EA CAR-T cells on the killing effect of NK cells.
Figure 13 shows the inhibitory effect of bbzg-PA CAR-T cells on the killing effect of T cells.

### Examples

### Example 1: Preparation of double-gene knockout CD7-UCAR T cells

Sequences encoding the following proteins were synthesized and sequentially cloned into pLVX vector (Public Protein/Plasmid Library (PPL), Cat. No.: PPL00157-4a): CD8α signal peptide (SEQ ID NO: 36), anti-CD7 scFv (SEQ ID NO: 12), CD8α hinge region (SEQ ID NO: 38), CD8α transmembrane region (SEQ ID NO: 22), 4-1BB co-stimulator domain (SEQ ID NO: 28), CD3ζ primary signaling domain (SEQ ID NO: 32) to obtain traditional bbz-CAR plasmids, and the correct insertion of the target sequence was confirmed by sequencing. The bbzg-CAR plasmid was obtained by the same method, and the only difference is that it also included the intracellular region of the γ chain (SEQ ID NO: 65) connected to the primary signaling domain of CD3ζ, wherein the intracellular region of the γ chain is located at the C-terminus of CD3ζ.

Three ml Opti-MEM (Gibco, Cat. No. 31985-070) was added to a sterile tube to dilute the above plasmid, and then packaging vector psPAX2 (Addgene, Cat. No. 12260) and envelope vector pMD2.G (Addgene, Cat. No. 12259) were added according to the ratio of plasmid : viral packaging vector: viral envelope vector = 4:2:1. Then, 120 µl X-treme GENE HP DNA transfection reagent (Roche, Cat. No. 06366236001) was added, mixed immediately, and incubated at room temperature for 15 min, and then the plasmid/vector/transfection reagent mixture was added dropwise to the culture flask of 293T cells. Viruses were collected at 24 hours and 48 hours, pooled, and ultracentrifuged (25000g, 4°C, 2.5 hours) to obtain concentrated bbz-CAR lentiviruses and bbzg-CAR lentiviruses.

T cells were activated with DynaBeads CD3/CD28 CTSTM (Gibco, Cat. No. 40203D), and were further cultured for 1 day at 37°C and 5% CO₂. Then the CRISPR/Cas9 system was used to knock out the TCR/CD3 components (specifically the TRAC gene) and the CD7 gene in wild-type T cells to obtain TCR/CD7 double knockout dKO-T cells. Wild-type T cells without gene knockout (i.e., NT cells) were used as control.

Using FITC Mouse Anti-Human CD3 (BD Pharmingen, Cat. No. 555916) antibody and PE mouse anti-human CD7 (biolegend, Cat. No. 395604), the gene editing efficiency of TCR and CD7 in the T cells was detected by flow cytometry, and the results are shown in Table 1.

**Table 1. Gene expression efficiency in the T cells**

| Name | TCR/CD3 | CD7 |
|---|---|---|
| dKO-T | 3.7% | 7.6% |
| NT | 99% | 96% |

It can be seen from Table 1 that the expression of related genes in the dKO-T cells prepared in the present disclosure is effectively suppressed or silenced.

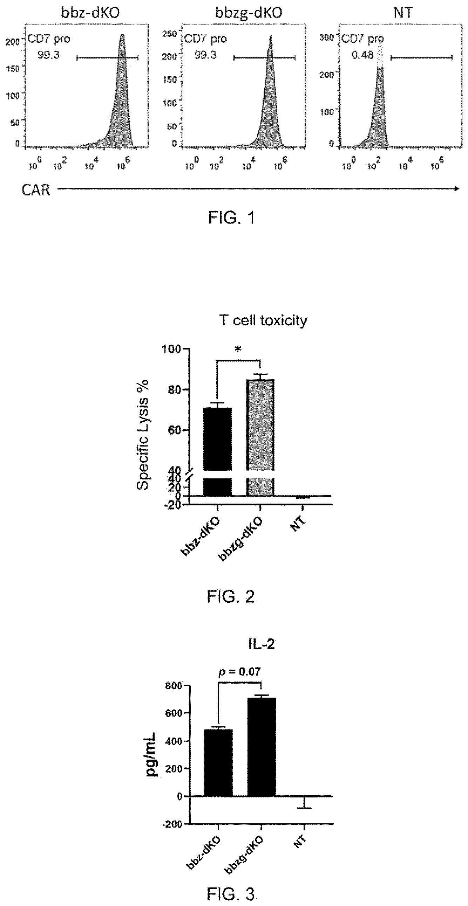

The concentrated lentiviruses were added to dKO-T cells to obtain two kinds of UCAR-T cells (i.e., bbz-dKO T cells and bbzg-dKO T cells). After 11 days of culture at 37°C and 5% CO₂, the expression level of scFv on the UCAR T cells was detected by flow cytometry using Biotin-SP (long spacer) AffiniPure Goat Anti-Mouse IgG, F(ab')₂ Fragment Specific (min X Hu, Bov, Hrs Sr Prot) (jackson immunoresearch, Cat. No. 115-065-072) as the primary antibody, APC Streptavidin (BD Pharmingen, Cat. No. 554067) or PE Streptavidin (BD Pharmingen, Cat. No. 554061) as the secondary antibody, and the results are shown in Figure 1.

It can be seen that the scFv in the UCAR-T cells prepared in this example can be effectively expressed, indicating that the addition of the intracellular region of the γ chain did not affect the surface expression of the CAR structure.

### Example 2. Killing effect of UCAR T cells on target cells and release of cytokines of UCAR T cells

### 2.1 The killing effect of UCAR T cells on target cells

In order to detect the killing ability of CAR-T cells on target cells, first Jurkat target cells carrying the fluorescein gene were plated into a 96-well plate at 1x10⁴/well, and then the two CAR T cells and NT cells were plated into the 96-well plate with an effector-to-target ratio of 2.5:1 for co-culture, and the fluorescence value was measured with a microplate reader after 8 hours. According to the calculation formula: (average fluorescence value of target cells - average fluorescence value of samples)/average fluorescence value of target cells × 100%, the killing efficiency was calculated, and the results are shown in Figure 2.

It can be seen that compared with NT, both bbz-dKO T cells and bbzg-dKO T cells have specific killing effect on target cells, and the killing effect of bbzg-dKO T cells of the present disclosure on target cells is significantly higher than that of traditional bbz-dKO T cells.

### 2.2 Cytokine release of UCAR-T cells

When T cells kill target cells, the number of target cells decreases and cytokines such as IL2 and IFN-γ are released at the same time. According to the following steps, enzyme-linked immunosorbent assay (ELISA) was used to measure the release level of cytokine IL2 when the UCAR T cells of the present disclosure kill target cells.

### (1) Collection of cell co-culture supernatant

Jurkat target cells were plated in a 96-well plate at a concentration of 1×10⁵/well, and then two UCAR T cells and NT cells (negative control) were co-cultured with the target cells at a ratio of 1:1, respectively, and cell co-culture supernatants were collected after 8 hours.

### (2) Detection of the secretion of IL2 in the supernatant by ELISA

A 96-well plate was coated with Purified anti-human IL-2 Antibody (Biolegend, Cat. No. 500302) as capture antibody and incubated overnight at 4°C, and then the antibody solution was removed. 250 µL of PBST (1XPBS containing 0.1% Tween) solution containing 2% BSA (sigma, Cat. No. V900933-1kg) was added, and incubated at 37°C for 2 hours. The plate was then washed 3 times with 250 µL PBST (1XPBS containing 0.1% Tween). 50 µL of cell co-culture supernatant or standard per well was added and incubated at 37°C for 1 h, then the plate was washed 3 times with 250 µL of PBST (1XPBS containing 0.1% Tween). Then, 50 µL Anti-IL-2 antibody as detection antibody was added to each well, and after incubation at 37°C for 1 hour, the plate was washed 3 times with 250 µL PBST (1XPBS containing 0.1% Tween). Then HRP Streptavidin (Biolegend, Cat. No. 405210) was added, incubated at 37°C for 30 minutes, and the supernatant was discarded. 250 µL PBST (1XPBS containing 0.1 % Tween) was added for washing 5 times. 50 µL of TMB substrate solution was added to each well. Reactions were allowed to occur at room temperature in the dark for 30 minutes, after which 50 µL of 1 mol/L H₂SO₄ was added to each well to stop the reaction. Within 30 minutes of stopping the reaction, a microplate reader was used to detect the absorbance at 450 nm, and the content of cytokines was calculated according to the standard curve (drawn according to the reading value and concentration of the standard), the results are shown in Figure 3.

It can be seen that the cytokine release of bbz-dKO T cells and bbzg-dKO T cells to target cells was significantly higher than that of the control NT group, and the release level of bbzg-dKO T cells was significantly higher than that of bbz-dKO T cells.

### Example 3 Preparation of triple-gene knockout CD7-UCAR T cells and verification of their functions

T cells were activated with DynaBeads CD3/CD28 CTSTM (Gibco, Cat. No. 40203D), and were further cultured for 1 day at 37°C and 5% CO₂. Then the CRISPR/Cas9 system was used to knock out TCR/CD3 components (specifically TRAC gene), CD7 gene and MHC class II related gene (specifically RFX5) in wild-type T cells to obtain TCR/CD7/RFX5 triple knockout tKO-T cells. Wild-type T cells without gene knockout (i.e., NT cells) were used as control.

Using FITC Mouse Anti-Human CD3 (BD Pharmingen, Cat. No. 555916) antibody, PE mouse anti-human CD7 (biolegend Cat. No. 395604) and APC anti-human DR, DP, DQ (biolegend, Cat. No. 361714) antibody, the gene editing efficiency of TCR/CD7/RFX5 in the T cells was detected by flow cytometry, and the results are shown in Table 2.

**Table 2. Gene expression efficiency in the T cells**

| Name | TCR/CD3 | CD7 | RFX5/MHC-II |
|---|---|---|---|
| tKO-T | 3.8% | 10.7% | 15.8% |
| NT | 99% | 92% | 92% |

It can be seen from Table 2 that the expression of related genes in the tKO-T cells prepared in the present disclosure is effectively suppressed or silenced.

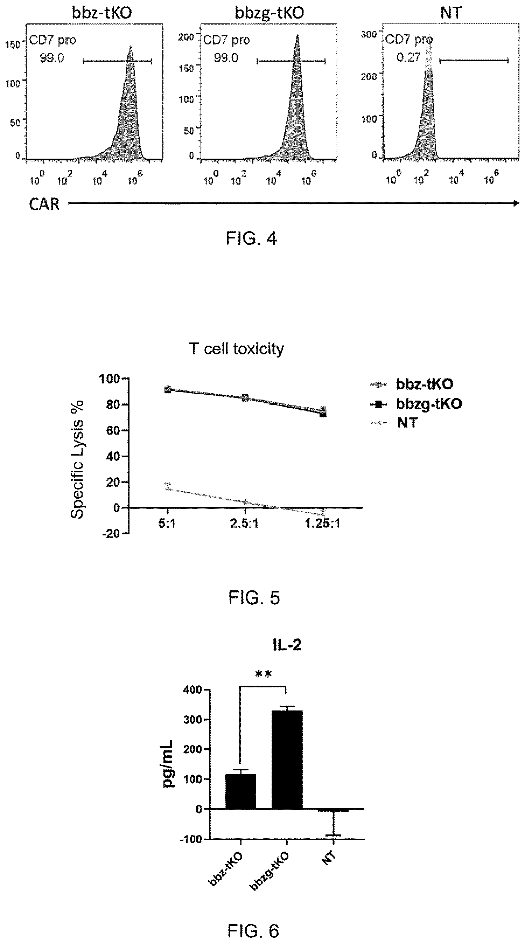

The bbz-CAR lentivirus and bbzg-CAR lentivirus prepared in Example 1 were added to tKO-T cells to obtain two kinds of UCAR-T cells (i.e., bbz-tKO T cells and bbzg-tKO T cells), and the expression level of scFv on UCAR T cells was detected by flow cytometry, and the results are shown in FIG. 4. It can be seen that all the scFvs in the UCAR T cells prepared in this example can be effectively expressed.

According to the method described in Example 2, the specific killing activity of UCAR-T cells on target cells (the effector-to-target ratio of 5:1, 2.5:1 and 1.25:1) and the release level of cytokine IL-2 were detected, and the results are shown in Figure 5 and Figure 6, respectively. The specific killing of target cells and cytokine release by bbz-tKO T cells and bbzg-tKO T cells were significantly higher than those in the control NT group, and the release level of bbzg-tKO T cells was higher than that of bbz-tKO T cells.

### Example 4. Preparation of dual-target UCAR-T cells and verification of their functions

Sequences encoding the following proteins were synthesized and cloned into pLVX vector (Public Protein/Plasmid Library (PPL), Cat. No.: PPL00157-4a): CD8α signal peptide (SEQ ID NO: 36), anti-CD7 scFv (SEQ ID NO: 12), connecting peptide (SEQ ID NO: 67), anti-CD19 scFv (SEQ ID NO: 58), CD8α hinge region (SEQ ID NO : 38), CD8α transmembrane region (SEQ ID NO: 22), 4-1BB intracellular region (SEQ ID NO: 28), CD3ζ intracellular signaling domain (SEQ ID NO: 32) and γ chain intracellular region (SEQ ID NO: 65), and the correct insertion of the target sequence was confirmed by sequencing.

According to the method of Example 1, the above plasmid vector was packaged into a lentivirus, and infected into tkO-T cells to obtain CAR7X19 T cells. Unmodified wild-type T cells were used as negative controls (NT).

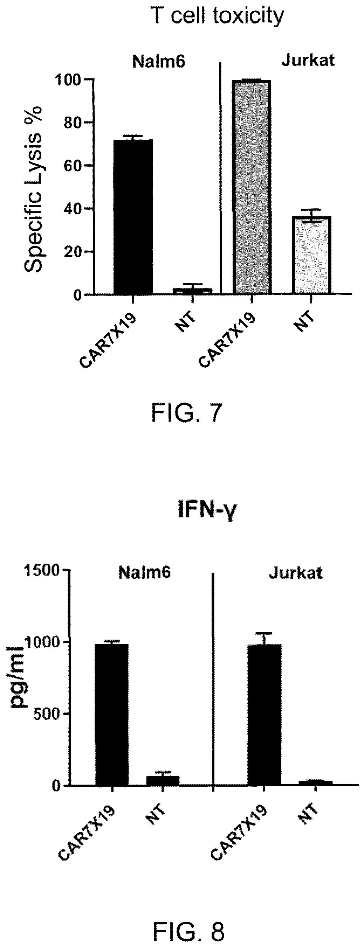

According to the method described in Example 2, the killing function of CAR7X19 T cells on target cells (Nalm6 and Jurkat) was detected, and the results are shown in Figure 7; the release level of cytokine IFN-γ was detected with Purified anti-human IFN-γ Antibody (Biolegend, Cat. No. 506502), and the results are shown in Figure 8.

It can be seen that compared with NT cells, CAR7X19 T cells had significantly increased specific killing activity and cytokine release level against both Nalm6 and Jurkat target cells.

### Example 5. Inhibitory effect of immunosuppressive molecules on NK cell killing

Immunosuppressive molecules No.1-14 were synthesized as shown in Table 3, and cloned into pLVX vector plasmid (Public Protein/Plasmid Library (PPL), Cat. No.: PPL00157-4a) according to the method described in Example 1, and then transfected into lentivirus and used to infect activated T cells to obtain T cells expressing immunosuppressive molecules. The CRISPR system was used to knock out TCR/CD3 components (specifically TRAC gene) and MHC-related genes (specifically B2M and RFX5) in cells expressing immunosuppressive molecule No.1-10, and knock out B2M gene in cells expressing immunosuppressive molecule No. 11-14, then flow cytometry was used to confirm that each gene was effectively knocked out to obtain UNKi-T cells.

**Table 3. Structure of immunosuppressive moleculars**

| No. | Name | Structure of immunosuppressive moleculars | | | | |
|---|---|---|---|---|---|---|
| | | Signal peptid e | Immune cell antigen binding region | Hinge region | Trans membr ane domain | Costimulat ory domain |
| 1 | KIRG4 | B2M | Anti-KIR scFv | lgG4 | CD8α | CD28 |
| 2 | LIR1-1 | B2M | Anti-LIR1 scFv-1 | lgG4 | CD8α | CD28 |
| 3 | LIR1-2 | CD8α | Anti-LIR1 scFv-2 | CD28 | CD28 | CD28 |
| 4 | Ecad0 | B2M | E-cadherin extracellular region | CD28 | CD28 | None |
| 5 | Ecad28 | B2M | E-cadherin extracellular region | CD28 | CD28 | CD28 |
| 6 | E0 | B2M | Presenting peptide + mutant B2M + HLA-E extracellular region | None | CD28 | None |
| 7 | E28 | B2M | Presenting peptide + mutant B2M + HLA-E extracellular region | None | CD28 | CD28 |
| 8 | G0 | B2M | Mutant B2M+ HLA-G extracellular region | None | CD28 | None |
| 9 | G28 | B2M | Mutant B2M+ HLA-G extracellular region | None | CD28 | CD28 |
| 10 | A28 | B2M | Anti-NKG2A-scFv | lgG4 | CD28 | CD28 |
| 11 | SC7G4 | B2M | Anti-SIGLEC7 scFv | lgG4 | CD8α | CD28 |
| 12 | SC7/SC9G 4 | B2M | Anti-SIGLEC7/SIGLEC9 scFv | IgG4 | CD8α | CD28 |
| 13 | K1G4 | B2M | Anti-KLRG1 scFv | IgG4 | CD8α | CD28 |
| 14 | P1 | PDL1 | PDL1 extracellular region | None | PDL1 | CD28 |

Wherein, the amino acid sequence of the anti-KIR scFv is set forth in SEQ ID NO: 71; the amino acid sequence of the anti-LIR1 scFv-1 is set forth in SEQ ID NO: 72; the amino acid sequence of the anti-LIR1 scFv-2 is set forth in SEQ ID NO: 73 ; the amino acid sequence of the extracellular region of E-cadherin is set forth in SEQ ID NO: 74; the amino acid sequence of the presenting peptide is set forth in SEQ ID NO: 75; the nucleic acid sequence of the mutant B2M is set forth in SEQ ID NO: 76, and the amino acid sequence is set forth in SEQ ID NO: 77; the amino acid sequence of the extracellular region of HLA-E is set forth in SEQ ID NO: 78; the amino acid sequence of HLA-G is set forth in SEQ ID NO: 79; the amino acid sequence of anti-NKG2A scFv is set forth in SEQ ID NO: 80; the amino acid sequence of anti-SIGLEC7 scFv is set forth in SEQ ID NO: 81; the amino acid sequence of anti-SIGLEC7/SIGLEC9 scFv is set forth in SEQ ID NO: 82; the amino acid sequence of anti-KLRG1 scFv is set forth in SEQ ID NO: 83; the amino acid sequence of PDL1 signal peptide is set forth in SEQ ID NO: 68; the amino acid sequence of PDL1 extracellular region is set forth in SEQ ID NO: 69; the amino acid sequence of PDL1 transmembrane region is set forth in SEQ ID NO: 70; the amino acid sequence of the IgG4 hinge region is set forth in SEQ ID NO: 42; the amino acid sequence of the CD28 hinge region is set forth in SEQ ID NO: 40; the amino acid sequence of the CD8α transmembrane region is set forth in SEQ ID NO: 22; the amino acid sequence of the CD28 transmembrane region is set forth in SEQ ID NO: 24; the amino acid sequence of the CD28 co-stimulatoryry domain is set forth in SEQ ID NO: 26; the amino acid sequence of the B2M signal peptide is set forth in SEQ ID NO: 34; the amino acid sequence of the CD8α signal peptide is set forth in SEQ ID NO: 36.

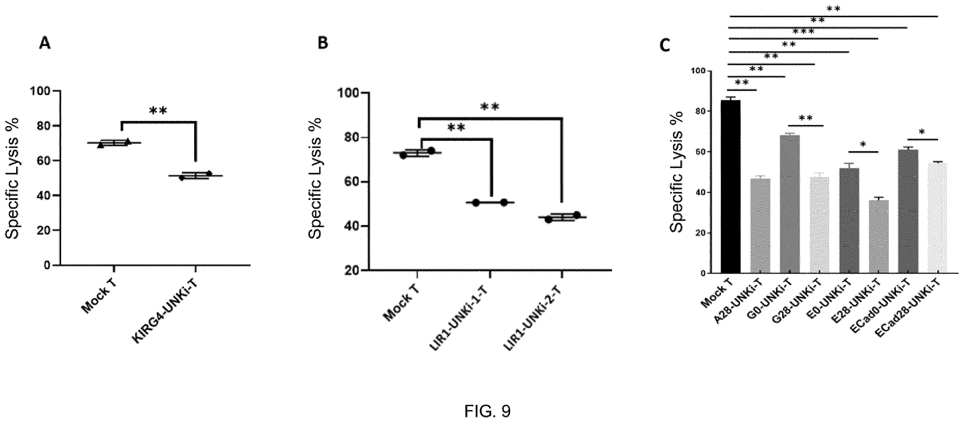

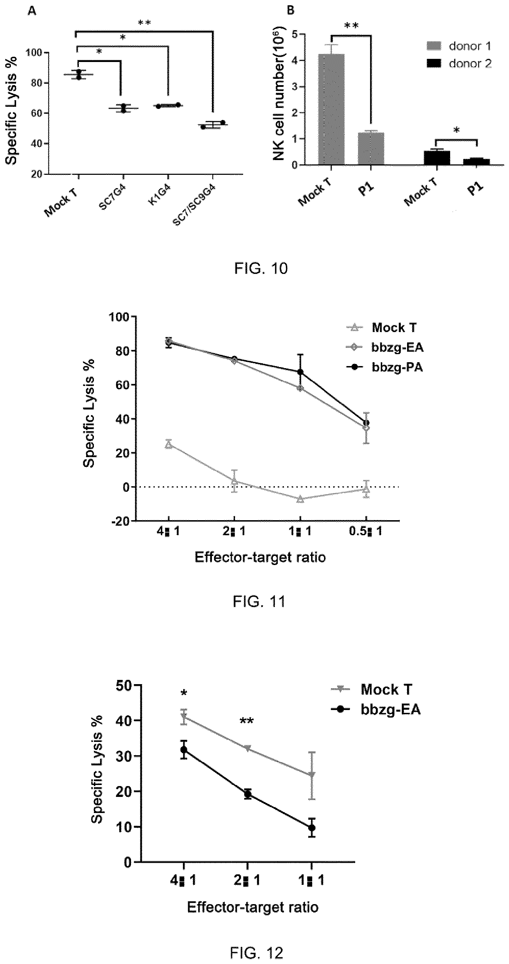

Then, the inhibitory effect of UNKi-T cells expressing immunosuppressive molecules No.1-13 on the killing effect of NK cells was detected according to the following method: the UNKi-T cells and Mock-T cells prepared in the present disclosure were labeled with Far-Red (invitrogen, Cat. No. : C34564). Then the labeled UNKi-T cells and NT cells were plated into 96-well plates at a concentration of 1×10⁴ cells/well, and NK92 cells (for UNKi-T cells expressing immunosuppressive molecules No. 3-10 and Mock T cells ) or NK92-KLRG1 cells (for UNKi-T cells expressing immunosuppressive molecules No.1-2 and 11-13, prepared by introducing KLRG1 gene into NK92 cells) for co-culture. After 16-18 hours, the proportion of T cells in the culture was detected by flow cytometry, and then the killing effect of NK cells on T cells was calculated. The results are shown in Figure 9 and Figure 10A.

The following method was used to detect the inhibitory effect of T cells expressing the immunosuppressive molecule No.14 on the killing effect of NK cells: T cells were cultured and treated with cytomitomycin C, and two allogeneic PBMCs (donor 1 and donor 2) were labeled with Far-Red, and co-cultured according to the ratio of T cells:PBMC=1:2. A half-media-change treatment was performed every 2-3 days. After 8 days, the cells were counted and stained with PE anti-human CD3 (manufacturer biolegend, Cat. No. : 317308) and FITC anti-human CD56 (manufacturer: biolegend, Cat. No. : 362546), and then the proportion of NK cell population was detected by flow cytometry. The number of NK cells was calculated by the total number of cells*the proportion of NK cell population, and the results are shown in Figure 10B.

It can be seen from Figure 9 and Figure 10 that, compared with T cells that do not express immunosuppressive molecules, UNKi-T cells expressing immunosuppressive molecules No. 1-14 significantly reduced the killing effect of NK cells on T cells. Moreover, compared with T cells (G0, E0, Ecad0) expressing only the antigen-binding region of immune cells and the transmembrane domain (i.e., not containing the co-stimulatory domain), the addition of co-stimulatory domains further significantly enhanced the inhibition effect of T cells on NK cell killing (G28, E28, ECad28, see Figure 9C). Therefore, the above immunosuppressive molecules significantly reduced the killing effect of NK cells on UNKi-T cells, thereby effectively reducing the risk of HvGD.

### Example 6. The killing activity of CAR-T cells expressing two kinds of immunosuppressive molecules and the inhibitory effect on the killing effect of immune cells

The bbzg-CAR plasmid prepared in Example 1 further contains the immunosuppressive molecules No.5 and 10 shown in Table 1 (connected by 2A peptide) to obtain the bbzg-EA plasmid; or further contains the immunosuppressive molecules No.14 and 10 (connected by 2A peptide ligation) to obtain the bbzg-PA plasmid. According to the method described in Example 1, the above two plasmids were packaged into viruses, and transfected into T cells in which the three genes of TCR/CD7/CIITA were knocked out to obtain a general-purpose CAR- T cells expressing bbzg-EA and bbzg-PA. T cells in which TCR/CD7/CIITA three genes were knocked out were used as control (Mock T cells).

According to the method described in Example 2, the killing effect of the above-mentioned universal CAR-T cells on Jurkat target cells was detected, and the results are shown in Figure 11. It can be seen that under various effector-to-target ratios, the universal CAR-T cells expressing bbzg-EA and bbzg-PA showed a strong killing effect on Jurkat target cells.

The inhibitory effect of the above-mentioned universal CAR-T cells expressing bbzg-EA on the killing effect of NK cells was detected by the following method: the bbzg-EA-CAR T cells and NT cells prepared in the present disclosure were labeled with Far-Red (invitrogen, Cat. No. : C34564). Then, the labeled bbzg-EA-CAR T cells and NT cells were plated into 96-well plates at a concentration of 1×10⁴ cells/well, and NK92 cells were added at an effector-to-target ratio of 4:1, 2:1 or 1:1 for co-culture. After 16-18 hours, the proportion of T cells in the culture was detected by flow cytometry, and then the killing rate of NK cells on T cells was calculated. The results are shown in Figure 12 . It can be seen that under different effector-to-target ratios, the universal CAR-T cells significantly inhibited the killing effect of NK cells on it.

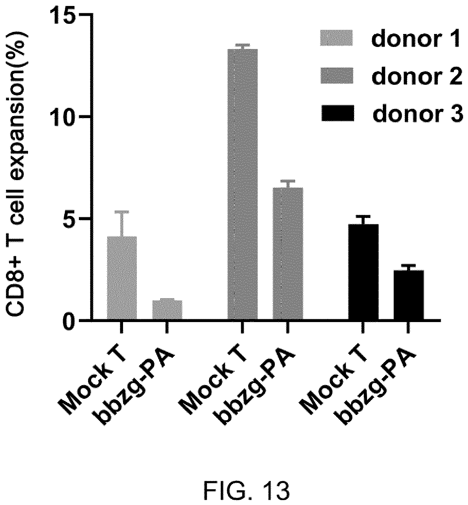

In addition, the inhibitory effect of the above-mentioned universal CAR-T cells expressing bbzg-PA on the killing effect on T cells was detected by the following method: the CAR-T cells and NT cells were cultured and treated with Cytomitomycin C, and at the same time, three allogeneic PBMCs (donor 1, donor 2 and donor 3) were labeled with Far-Red, and co-cultured according to the ratio of T cells: PBMC = 1:2. A half-media-change treatment was performed every 2-3 days. After 8 days, the cells were counted and stained with PE anti-human CD8 (manufacturer biolegend, Cat. No. : 344706), and then the proportion of T cell population was detected by flow cytometry. The number of T cells was calculated by the total number of cells * the proportion of the T cell population. The result is shown in Figure 13. It can be seen that the universal CAR-T cells expressing the combination of two immunosuppressive molecules significantly inhibited the killing effect of T cells.

In summary, immunosuppressive molecules do not affect the killing activity of CAR-T cells, and can significantly inhibit the killing effect of immune cells (such as NK cells, T cells, etc.) on CAR-T cells.

It should be noted that the above-mentioned are merely for preferred examples of the present disclosure and not used to limit the present disclosure. For one skilled in the art, various modifications and changes may be made to the present disclosure. Those skilled in the art should understand that any amendments, equivalent replacements, improvements, and so on, made within the spirit and principle of the present disclosure, should be covered within the scope of protection of the present disclosure.

## Claims

1. A chimeric antigen receptor, comprising an antigen binding region, a transmembrane domain and an intracellular signaling region, **characterized in that** the antigen binding region comprises an antibody specifically targeting CD7, and the intracellular signaling region consists of at least one co-stimulatory domain, a primary signaling domain, and a γc chain or intracellular region thereof.

2. The chimeric antigen receptor according to claim 1, **characterized in that** the antibody is selected from the group consisting of IgG, Fab, Fab', F(ab')₂, Fd, Fd', Fv, scFv, sdFv, linear antibody, single domain antibody, nanobody and diabody.

3. The chimeric antigen receptor according to claim 1, **characterized in that** the transmembrane domain is a transmembrane domain of a protein selected from the group consisting of: TCRα chain, TCRβ chain, TCRγ chain, TCRδ chain, CD3ζ subunit, CD3ε subunit, a CD3γ subunit, a CD3δ subunit, CD45, CD4, CD5, CD8, CD9, CD16, CD22, CD33, CD28, CD37, CD64, CD80, CD86, CD134, CD137, and CD154.

4. The chimeric antigen receptor according to claim 1, **characterized in that** the primary signaling domain is a signaling domain of a protein selected from the group consisting of: FcRγ, FcRβ, CD3γ, CD3δ, CD3ε, CD3ζ, CD22, CD79a, CD79b, and CD66d.

5. The chimeric antigen receptor according to claim 1, **characterized in that** the at least one co-stimulatory domain is selected from the group consisting of co-stimulatory signaling domains of proteins: TLR1, TLR2, TLR3, TLR4, TLR5, TLR6, TLR7, TLR8, TLR9, TLR10, CARD11, CD2, CD7, CD8, CD18, CD27, CD28, CD30, CD40, CD54, CD83, CD134, CD137, CD150, CD152, CD223, CD270, CD272, CD273, CD274, CD276, CD278, CD357, DAP10, LAT, NKG2C, SLP76, LIGHT, TRIM, CD94, LTB, and ZAP70.

6. The chimeric antigen receptor according to claim 1, **characterized in that** the γc chain has at least 95% sequence identity to SEQ ID NO: 63 or 65.

7. The chimeric antigen receptor according to claim 1, **characterized in that** the antibody targeting CD7 comprises CDR-L1 as set forth in SEQ ID NO: 1, CDR-L2 as set forth in SEQ ID NO: 2, CDR-L3 as set forth in SEQ ID NO: 3, CDR-H1 as set forth in SEQ ID NO: 4, CDR-H2 as set forth in SEQ ID NO: 5 and CDR-H3 as set forth in SEQ ID NO: 6.

8. The chimeric antigen receptor according to claim 7, **characterized in that** the antibody targeting CD7 comprises a light chain variable region having at least 95% sequence identity to an amino acid sequence selected from the group consisting of SEQ ID NOs: 7, 10, 13, 16 and 19, and a heavy chain variable region having at least 95% sequence identity to an amino acid sequence selected from the group consisting of SEQ ID NOs: 8, 11, 14, 17 and 20.

9. The chimeric antigen receptor according to claim 1, **characterized in that** the antigen binding region further comprises an antibody targeting a second antigen, wherein the second antigen is selected from the group consisting of: TSHR, CD19, CD123, CD22, BAFF-R, CD30, CD171, CS-1, CLL-1, CD33, EGFRvIII, GD2, GD3, BCMA, GPRC5D, Tn Ag, PSMA, ROR1, FLT3, FAP, TAG72, CD38 , CD44v6, CEA, EPCAM, B7H3, KIT, IL-13Ra2, mesothelin, IL-1 1Ra, PSCA, PRSS21, VEGFR2, LewisY, CD24, PDGFR-β, SSEA-4, CD20, Folate receptor α, ERBB2 (Her2/neu), MUC1, EGFR, NCAM, Claudin18.2, Prostase, PAP, ELF2M, Ephrin B2, IGF-I receptor, CAlX, LMP2, gploo, bcr-ab1, tyrosinase, EphA2, Fucosyl GM1, sLe, GM3, TGS5, HMWMAA, o-acetyl-GD2, Folate receptor β, TEM1/CD248, TEM7R, CLDN6, GPRC5D, CXORF61, CD97, CD 179a, ALK, polysialic acid, PLAC1, GloboH, NY-BR-1, UPK2, HAVCR1, ADRB3, PANX3, GPR20, LY6K, OR51E2, TARP, WT1, NY-ESO-1, LAGE-1a, MAGE-A1, legumain, HPV E6, E7, MAGE A1, ETV6-AML, sperm protein 17, XAGE1, Tie 2, MAD-CT-1, MAD-CT-2, Fos associated antigen 1, p53, p53 mutant, prostate specific protein, survivin and telomerase, PCTA-1/Galectin 8, MelanA/MART1, Ras mutant, hTERT, sarcoma translocation breakpoint, ML-IAP, ERG (TMPRSS2 ETS fusion gene), NA17, PAX3, androgen receptor, Cyclin B1, MYCN, RhoC, TRP-2, CYP1B 1, BORIS, SART3, PAX5, OY-TES 1, LCK, AKAP-4, SSX2, RAGE-1, human telomerase reverse transcriptase, RU1, RU2, intestinal tract carboxylesterase, mut hsp70-2, CD79a, CD79b, CD72, LAIR1, FCAR, LILRA2, CD300LF, CLEC12A, BST2, EMR2, LY75, GPC3, FCRL5, IGLL1, PD1, PDL1, PDL2, TGFβ, APRIL, NKG2D and any combination thereof.

10. The chimeric antigen receptor according to claim 9, **characterized in that** the antibody targeting a second antigen is an antibody targeting CD19 comprising:
(1) CDR-L1 as set forth in SEQ ID NO: 44, CDR-L2 as set forth in SEQ ID NO: 45, CDR-L3 as set forth in SEQ ID NO: 46, CDR-H1 as set forth in SEQ ID NO: 47, CDR-H2 as set forth in SEQ ID NO: 48 and CDR-H3 as set forth in SEQ ID NO: 49; or
(2) CDR-L1 as set forth in SEQ ID NO: 50, CDR-L2 as set forth in SEQ ID NO: 51, CDR-L3 as set forth in SEQ ID NO: 52, CDR-H1 as set forth in SEQ ID NO: 53, CDR-H2 as set forth in SEQ ID NO: 54 and CDR-H3 as set forth in SEQ ID NO: 55.

11. The chimeric antigen receptor according to claim 10, **characterized in that** the antibody targeting CD19 comprises a light chain variable region having at least 95% sequence identity to the amino acid sequence as set forth in SEQ ID NO: 56 or 59 and a heavy chain variable region having at least 95% sequence identity to the amino acid sequence as set forth in SEQ ID NO: 57 or 60.

12. A nucleic acid encoding the chimeric antigen receptor according to any one of claims 1-11.

13. A vector comprising the nucleic acid according to claim 12.

14. The vector according to claim 13, **characterized in that** the vector is a linear nucleic acid molecule, a plasmid, a retrovirus, a lentivirus, an adenovirus, a vaccinia virus, a Rous sarcoma virus (RSV), a polyoma virus, an adeno-associated virus (AAV), a bacteriophage, a cosmid or an artificial chromosome.

15. An engineered immune cell, comprising the chimeric antigen receptor according to any one of claims 1-11, the nucleic acid according to claim 12, or the vector according to claim 13 or 14.

16. The engineered immune cell according to claim 15, **characterized in that** the immune cell is selected from the group consisting of a T cell, a macrophage, a dendritic cell, a monocyte, a NK cell and a NKT cell.

17. The engineered immune cell according to claim 16, **characterized in that** the immune cell is a T cell selected from the group consisting of: a CD4+/CD8+ double-positive T cell, a CD4+ helper T cell, a CD8+ T cell, a tumor infiltrating cell, a memory T cell, a naive T cell, a CD4-CD8-T cell, a regulatory T cell, a γδ-T cell, and an αβ-T cell.

18. The engineered immune cell according to any one of claims 15-17, **characterized in that** the immune cell comprises suppressed or silenced expression of endogenous CD7.

19. The engineered immune cell according to any one of claims 15-18, **characterized in that** the immune cell comprises suppressed or silenced expression of at least one TCR/CD3 gene.

20. The engineered immune cell according to any one of claims 15-19, **characterized in that** the immune cell comprises suppressed or silenced expression of at least one MHC class II related gene.

21. The engineered immune cell according to any one of claims 19-20, **characterized in that** the TCR/CD3 gene is selected from the group consisting of TRAC, TRBC, CD3γ, CD3δ, CD3ε, CD3ζ, and a combination thereof.

22. The engineered immune cell according to claim 20, **characterized in that** the MHC class II related gene is selected from the group consisting of: HLA-DPA, HLA-DQ, HLA-DRA, RFX5, RFXAP, RFXANK, CIITA, and a combination thereof.

23. The engineered immune cell according to claim 22, **characterized in that** the immune cell comprises suppressed or silenced expression of endogenous CD7, at least one TCR/CD3 gene selected from the group consisting of TRAC and TRBC, and at least one MHC class II related gene selected from the group consisting of RFX5, RFXAP, RFXANK and CIITA.

24. The engineered immune cell according to any one of claims 15-23, further expressing an immunosuppressive molecule, wherein the immunosuppressive molecule comprises one or more immune cell antigen binding regions, a transmembrane domain and at least one co-stimulatoryry domain.

25. The engineered immune cell of claim 24, **characterized in that** the immunosuppressive molecule does not comprise a primary signaling domain.

26. The engineered immune cell of claim 24, **characterized in that** the immune cell antigen binding region is an antibody targeting an immune cell antigen, a ligand of an immune cell antigen, or a combination thereof.

27. The engineered immune cell according to claim 24, **characterized in that** the immune cell antigen is selected from the group consisting of NKG2A, NKG2B, NKG2C, NKG2D, NKG2E, NKP46, LIR1, LIR2, LIR3, LIR5, LIR8, PD-1, TIGIT, TIM3, LAG3, KIR, CEACAM1, LAIR1, SIGLEC7, SIGLCE9, KLRG1, CD2, CD3, CD4, CD5, CD8, CD16a, CD16b, CD25, CD27, CD28, CD30, CD38, CD45, CD48, CD50, CD52, CD56, CD57 , CD62L, CD69, CD94, CD96, CD100, CD102, CD122, CD127, CD132, CD137, CD160, CD161, CD178, CD218, CD226, CD244, CD279, CD305, CD337, CS1, and a combination thereof.

28. The engineered immune cell according to claim 26, **characterized in that** the ligand of the immune cell antigen is selected from the group consisting of HLA-E, HLA-F, HLA-G, cadherin (such as E-cadherin, N-cadherin, R-cadherin), collagen, OCIL, sialic acid, PD-L1, PD-L2, CD155, CTLA4, CD112, CD113, Gal-9, FGL1 and extracellular region thereof.

29. A pharmaceutical composition comprising the chimeric antigen receptor according to any one of claims 1-11, the nucleic acid according to claim 12, the vector according to claim 13 or 14, or the engineered immune cell according to any one of claims 15-28, and one or more pharmaceutically acceptable excipients.

30. Use of the chimeric antigen receptor according to any one of claims 1-11, the nucleic acid according to claim 12, the vector according to claim 13 or 14, the engineered immune cell according to any one of claims 15-28, or the pharmaceutical composition according to claim 29 in the preparation of a medicine for treating a disease associated with CD7 expression.

31. The use according to claim 30, **characterized in that** the disease associated with CD7 expression is selected from the group consisting of: acute lymphoblastic leukemia, acute myeloid leukemia, chronic myelocytic leukemia, chronic lymphocytic leukemia, chronic myelogenous leukemia, T-lymphoblastic lymphoma, non-Hodgkin's lymphoma, peripheral T-cell lymphoma, extranodal NK/T-cell lymphoma, γδ T-cell lymphoma, and early T-cell precursor lymphoblastic leukemia.
